# EUROPEAN PATENT SPECIFICATION

(11) **EP 2 113 210 B1**
(45) Date of publication and mention of the grant of the patent: **09.03.2016**
(21) Application number: 09004452.0
(22) Date of filing: 27.03.2009
(51) Int. Cl.: A61B 17/32, A61B 17/29, A61B 90/00

(54) **Surgical operating apparatus**
Chirurgische Operationsvorrichtung
Appareil pour opération chirurgicale

(30) Priority: 28.04.2008 US 110807
(43) Date of publication of application: 04.11.2009
(73) Proprietor: Olympus Corporation, Shibuya-ku Tokyo (JP)
(72) Inventor: Yachi, Chie, Hachioji-shi Tokyo 192-8512 (JP); Masuda, Shinya, Hachioji-shi Tokyo 192-8512 (JP)
(74) Representative: von Hellfeld, Axel

(56) References cited:
- EP-A1- 1 870 045
- WO-A2-2006/042210
- WO-A2-2007/143439

## Description

The present invention relates to a surgical operating apparatus by which a treatment such as incision, resection, and coagulation is executed by transmitting at least one of ultrasonic vibration and high-frequency waves to the probe distal end section in a state where a living tissue is grasped between a probe distal end section arranged at a distal end part of a vibration transmission member for transmitting ultrasonic vibration and a jaw supported so as to be openable/closable with respect to the probe distal end section.

In general, as an example of an ultrasonic treatment apparatus for executing a treatment such as incision, resection, and coagulation by utilizing ultrasonic waves, an ultrasonic treatment instrument described in, for example, Jpn. Pat. Appln. KOKAI Publication No. 11-178833 (Pat. Document 1) is disclosed.

In this apparatus, a operation section on the hand side is coupled to a proximal end part of an elongated insertion section. This operation section is provided with an ultrasonic transducer for generating ultrasonic vibration. A distal end part of the insertion section is provided with a treatment section for treating a living tissue.

The insertion section includes an elongated sheath having a tubular shape. A stick-like vibrations transmission member (probe) is inserted into the sheath. The proximal end part of the vibration transmission member is detachably connected to the ultrasonic transducer through a threaded type coupling section. Further, ultrasonic vibration generated by the ultrasonic transducer is transmitted to the probe distal end section on the distal end side of the vibration transmission member.

The treatment section is provided with a jaw so as to be opposed to the probe distal end section. The proximal end part of the jaw is turnably supported at a distal end part of the sheath through a support shaft. A drive shaft for driving the jaw is inserted into the sheath so that it can be advanced or retreated in the axial direction. A jaw main body is coupled to a distal end part of the drive shaft through a coupling pin.

Further, the operation section is provided with a operation handle, and a power transmission mechanism for converting the operation of the operation handle into the advancing/retreating operation of the drive shaft in the axial direction. The operation handle includes a fixed handle and a movable handle that can be opened/closed with respect to the fixed handle. The power transmission mechanism includes a slider section which is advanced/retreated in the central axis direction of the vibration transmission member in accordance with the operation of the movable handle. A proximal end part of the drive shaft is fixed to a distal end of the slider section.

Further, concomitantly with the operation of the movable handle, the drive shaft is driven to be advanced/retreated in the axial direction through the slider section, and the jaw is operated to be opened/closed with respect to the probe distal end section concomitantly with the operation of the drive shaft.

At this time, the living tissue is grasped between the probe distal end section and the jaw concomitantly with the closing operation of the jaw. In this state, the ultrasonic vibration from the ultrasonic transducer is transmitted to the probe distal end section of the treatment section side through the vibration transmission member, whereby a treatment such as incision, resection, or coagulation of the living tissue is performed by utilizing the ultrasonic waves.

Further, in Jpn. Pat. Appln. KOKAI Publication No. 2004-129871 (Pat. Document 2), an ultrasonic treatment apparatus provided with a operation force adjustment section for adjusting the operation force of the movable handle is disclosed. Here, a coil spring or the like is incorporated in the slider section at a predetermined compression amount (equipped load). Further, when force exceeding the set value is applied to exceed the equipped load, the coil spring of the slider section is compressed, and the operation force of the movable handle is transmitted to the drive shaft through the slider section. As a result of this, the drive shaft is driven to be pushed in the axial direction, and the jaw is operated to be closed with respect to the probe distal end section concomitantly with the operation of the drive shaft.

By the way, the operation force of the movable handle differs between individuals according to the users. Further, when the operation force of the movable handle is weak, the grasping force at the time when the living tissue is grasped between the probe distal end section and the jaw becomes small. In this case, there is the possibility of the desired performance of the treatment instrument being not exerted. Conversely, when the operation force of the movable handle is strong, the grasping force at the time when the living tissue is grasped between the probe distal end section and the jaw becomes large. When the grasping force at the time when the living tissue is grasped between the probe distal end section and the jaw is varied as described above, the treating capability at the time when the incision, resection, or coagulation of the living tissue is performed by the ultrasonic vibration is also varied.

In the apparatus of Pat. Document 1, a stopper for controlling the advance/retreat amount of the slider section at the time when an operation for closing the jaw with respect to the probe distal end section is performed is shown. Further, when the slider section is brought into contact with the stopper, the subsequent movement of the slider section is restrained, whereby the operation amount of the jaw at the time when the operation for closing the jaw is performed is limited. However, in this case, until the slider section is brought into contact with the stopper, it cannot be prevented from occurring that the operation force of the movable handle is varied depending on the users. Thus, there is the possibility of the treating capability at the time of performing the treatment such as incision, resection, or coagulation of the living tissue by utilizing the ultrasonic vibration being varied.

In the apparatus of Pat. Document 2, two coil springs which resist the movement of the slider section are provided. In a state where the movable handle is at the opened position, only one of the coil springs is in contact with the slider section, and the other coil spring is held at a position separate from the slider section. Thus, when the movable handle is operated to be closed, one of the coil springs exerts the resistance force on the operation force of the movable handle since the beginning. Further, when the movement amount of the slider section exceeds a predetermined amount, the other coil spring is also brought into contact with the slider section. Thus, when the movement amount of the slider section exceeds the predetermined amount, the resistance force of the two coil springs is exerted on the operation force of the movable handle at a time. As a result of this, at the point of time at which the other coil spring is brought into contact with the slider section, it is possible to recognize a state where the slider section has been moved by a certain amount by a change in the resistance force exerted on the operation force of the movable handle.

However, in this case, a change in the resistance force exerted on the operation force of the movable handle is sensuously recognized by the user, and hence when the operation speed of the movable handle is low, there is the possibility a change in the resistance force exerted on the operation force of the movable handle being not perceived. Hence, there is the possibility that the variation in the treating capability at the time when the incision, resection, or coagulation of the living tissue is performed by the ultrasonic vibration cannot be securely prevented. Furthermore, in this case, in a region in which the resistance force of the two coil springs is simultaneously exerted during the operation of the movable handle, the operation force of the movable handle becomes heavier than in the region in which the resistance force of one coil spring is exerted. Thus, there is the possibility of the user becoming liable to be tired.

EP 1 870 045 A1 discloses an ultrasonic surgical instrument comprising an ultrasonic probe which is inserted into an actuator tube housed within an outer tube. The ultrasonic probe is positioned at a predetermined distance from the actuator tube. The ultrasonic surgical instrument further comprises a deflection detection circuit for detecting a deflection of the ultrasonic probe. If the ultrasonic probe is overstressed, the probe contacts the actuator tube, thereby closing the detection circuit and activating a visual alarm indicator.

The present invention has been contrived by paying attention to these circumstances, and an object thereof is to provide a surgical operating apparatus capable of preventing the operation force of the movable handle from being varied depending on the user, preventing the treating capability at the time when the incision, resection, or coagulation of the living tissue is performed by the ultrasonic vibration from being varied, and performing a stable operation.

According to the present invention, there is provided a surgical operating apparatus defined in claim 1. The surgical operating apparatus comprises: a sheath provided with a distal end part and a proximal end part; an apparatus main body to be coupled to the proximal end part of the sheath; a probe which is provided with a distal end part and a proximal end part, is inserted into the sheath, and transmits ultrasonic vibration from the proximal end part side to the distal end part side; a jaw which is turnably supported at the distal end part of the sheath, and is operated to be opened or closed between a closed position at which the jaw is engaged with the distal end part of the probe, and an opened position at which the jaw is separated from the distal end part of the probe; a handle which is provided on the apparatus main body, and operates the opening/closing operation of the jaw; a slider section which is provided in the apparatus main body, and is advanced/retreated to be moved in a central axis direction of the probe between a first movement position corresponding to the opened position of the jaw and a second movement position corresponding to the closed position of the jaw in accordance with the operation of the handle; and a notification mechanism for notifying of a state where the slider section has moved by an amount equal to or larger than a set amount on the way thereof from the first movement position to the second movement position.

Preferably, the notification mechanism includes a notification section which performs notification by means of at least one of a sound and a feeling of a click.

Preferably, the notification section includes a cylindrical body that is fixed to the side of a guide member for guiding the movement of the slider section, and is provided with a step section on an outer circumferential surface at which a diameter thereof is changed at a position corresponding to the set amount in a movement direction of the slider section, and a leaf spring member that is provided with two end parts, one end part of which is fixed to the slider section, and the other end part of which is held on the outer circumferential surface of the cylindrical body in a state where the other end part is in sliding contact with the outer circumferential surface of the cylindrical body, and at the time of the movement of the slider section, when the leaf spring member passes the step section, the other end part of the leaf spring member falls over the step section, and generates a knocking sound by knocking the circumferential wall surface on the lower side of the step section.

Preferably, the notification section includes a sound generation member which is provided on a guide surface for guiding the movement of the slider section when the slider section is moved, and generates a sound by being brought into contact with the slider section.

Preferably, the set amount is set at a position corresponding to a operation amount of the handle, the operation amount enabling the appropriate ultrasonic treating capability to be exerted when the living tissue is grasped between the jaw and the distal end part of the probe.

Preferably, the ultrasonic treating capability is at least one of coagulation and incision.

According to another aspect of the present disclosure, there is provided a surgical operating apparatus comprising: a sheath provided with a distal end part and a proximal end part; an apparatus main body to be coupled to the proximal end part of the sheath; a probe which is provided with a distal end part and a proximal end part, is inserted into the sheath, and transmits ultrasonic vibration from the proximal end part side to the distal end part side; a jaw which is turnably supported at the distal end part of the sheath, and is operated to be opened or closed between a closed position at which the jaw is engaged with the distal end part of the probe, and an opened position at which the jaw is separated from the distal end part of the probe; a handle which is provided on the apparatus main body, and operates the opening/closing operation of the jaw; a slider section which is provided in the apparatus main body, and is advanced/retreated to be moved in a central axis direction of the probe between a first movement position corresponding to the opened position of the jaw and a second movement position corresponding to the closed position of the jaw in accordance with the operation of the handle; and notification means for notifying of a state where the slider section has moved by an amount equal to or larger than a set amount on the way thereof from the first movement position to the second movement position.

The invention can be more fully understood from the following detailed description when taken in conjunction with the accompanying drawings, in which:
FIG. 1 is a perspective view showing a schematic configuration of an entire hand piece of a surgical operating apparatus according to a first embodiment of a present invention.
FIG. 2 is a perspective view showing a disassembled state in which a coupling part of an assembly unit of a hand piece of the surgical operating apparatus according to the first embodiment is detached.
FIG. 3 is a perspective view showing an external appearance of a handle unit of the surgical operating apparatus according to the first embodiment.
FIG. 4A is a longitudinal cross-sectional view showing a coupling state of the handle unit and a transducer unit of the surgical operating apparatus according to the first embodiment.
FIG. 4B is a cross-sectional view taken along line L4B-L4B of FIG. 4A.
FIG. 5A is a plan view showing a probe unit of the surgical operating apparatus according to the first embodiment.
FIG. 5B is a cross-sectional view taken along line L5B-L5B of FIG. 5A.
FIG. 6 is a longitudinal cross-sectional view of a sheath unit according to the surgical operating apparatus of the first embodiment.
FIG. 7 is a longitudinal cross-sectional view showing a coupling state of a jaw and a drive pipe of the surgical operating apparatus according to the first embodiment.
FIG. 8 is a cross-sectional view taken along line L8-L8 of FIG. 7.
FIG. 9 is a plan view showing a surface of the jaw of the surgical operating apparatus according to the first embodiment at which the jaw is opposed to a probe distal end section.
FIG. 10 is a cross-sectional taken at a cross-sectional position of line L10-L10 showing a state where a part between the jaw and the probe of the surgical operating apparatus according to the first embodiment is closed.
FIG. 11 is a longitudinal cross-sectional view showing a proximal end part of the sheath unit of the surgical operating apparatus according to the first embodiment.
FIG. 12 is a cross-sectional view taken along line L12-L12 of FIG. 11.
FIG. 13 is a cross-sectional view taken along line L13-L13 of FIG. 11.
FIG. 14 is a longitudinal cross-sectional view showing a knob member of the surgical operating apparatus according to the first embodiment in a state before assembling.
FIG. 15 is a longitudinal cross-sectional view showing a guide cylindrical body of the sheath unit of the surgical operating apparatus according to the first embodiment.
FIG. 16 is a front view showing a proximal end part of the guide cylindrical body of the sheath unit of the surgical operating apparatus according to the first embodiment.
FIG. 17 is a longitudinal cross-sectional view showing a main part of a fixed handle of the surgical operating apparatus according to the first embodiment in a state before assembling.
FIG. 18 is a longitudinal cross-sectional view showing an internal structure of the handle unit of the surgical operating apparatus according to the first embodiment.
FIG. 19 is a cross-sectional view taken along line L19-L19 of FIG. 18.
FIG. 20 is a cross-sectional view taken along line L20-L20 of FIG. 18.
FIG. 21 is a cross-sectional view taken along line L21-L21 of FIG. 18.
FIG. 22A is a longitudinal cross-sectional view showing the handle unit and the sheath unit of the surgical operating apparatus according to the first embodiment in a state before engagement.
FIG. 22B is a longitudinal cross-sectional view showing the handle unit and the sheath unit of the surgical operating apparatus according to the first embodiment in a state after engagement.
FIG. 23 is a plan view of a main part showing a notification mechanism of the hand piece of the surgical operating apparatus according to the first embodiment.
FIG. 24 is a perspective view showing a guide cylindrical body of the notification mechanism of the surgical operating apparatus according to the first embodiment.
FIG. 25 is a perspective view showing a leaf spring member of the notification mechanism of the surgical operating apparatus according to the first embodiment.
FIG. 26 is a longitudinal cross-sectional view of a main part for explaining a movement of the leaf spring member of the notification mechanism of the surgical operating apparatus according to the first embodiment.
FIG. 27 is an explanatory view for explaining a deformed state of the coil spring observed when a slider member of the surgical operating apparatus according to the first embodiment is operated.
FIG. 28 is a longitudinal cross-sectional view showing an internal structure of a handle unit of a second type different from the hand piece of the surgical operating apparatus according to the first embodiment.
FIG. 29 is a cross-sectional view taken along line L29-L29 of FIG. 28.
FIG. 30 is a longitudinal cross-sectional view of a sheath unit of the second type different from the surgical operating apparatus according to the first embodiment.
FIG. 31 is a longitudinal cross-sectional view showing a guide cylindrical body of a sheath unit of the second type different from the surgical operating apparatus according to the first embodiment.
FIG. 32 is a front view showing a proximal end part of the guide cylindrical body of the sheath unit of the second type different from the surgical operating apparatus according to the first embodiment.
FIG. 33 is a longitudinal cross-sectional view showing an attached state of a notification mechanism of a surgical operating apparatus according to a second embodiment of the present invention.
FIG. 34 is a longitudinal cross-sectional view showing an attached state of a sound generation section of the notification mechanism of the surgical operating apparatus according to the second embodiment.
FIG. 35 is a plan view showing the sound generation section of the notification mechanism of the surgical operating apparatus according to the second embodiment.
FIG. 36 is a longitudinal cross-sectional view showing the notification mechanism of the surgical operating apparatus according to the second embodiment.
FIG. 37 is a perspective view showing a handle unit of a hand piece of a surgical operating apparatus according to a third embodiment of the present invention.
FIG. 38 is a longitudinal cross-sectional view showing the handle unit of the hand piece of the surgical operating apparatus according to the third embodiment.
FIG. 39 is a longitudinal cross-sectional view of a main part showing a state after a fixed handle of the handle unit of the hand piece of the surgical operating apparatus according to the third embodiment is assembled.
FIG. 40 is a longitudinal cross-sectional view of a main part showing a state before the fixed handle of the handle unit of the hand piece of the surgical operating apparatus according to the third embodiment is assembled.

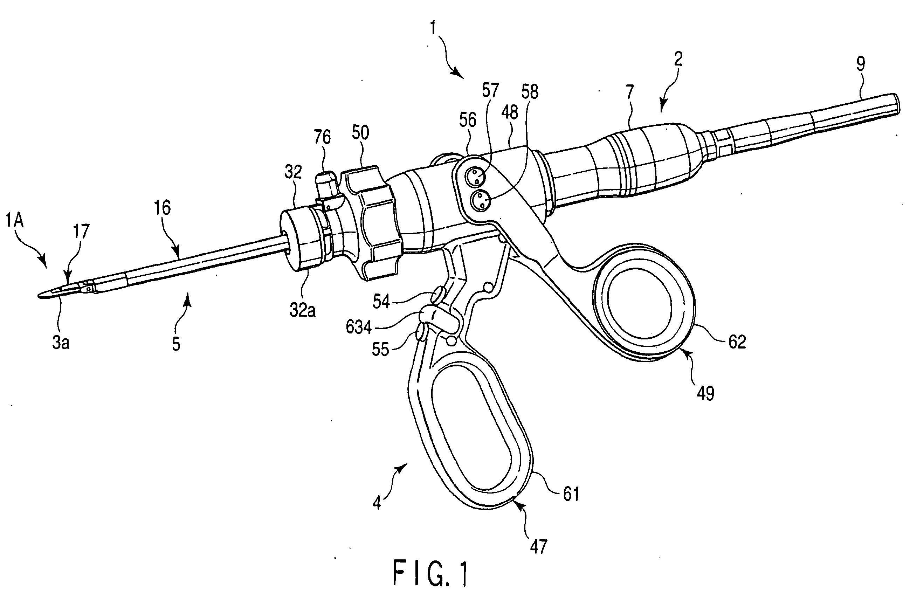

A first embodiment of the present invention will be described below with reference to FIGS. 1 to 27. FIG. 1 shows a schematic configuration of an entire hand piece 1 of an ultrasonic treatment apparatus which is a surgical operating apparatus of the first embodiment. The ultrasonic treatment apparatus of this embodiment is an ultrasonic coagulation-incision treatment apparatus. This ultrasonic coagulation-incision treatment apparatus can perform a treatment such as incision, resection, or coagulation of a living tissue by utilizing ultrasonic waves, as well as a treatment by utilizing high-frequency waves.

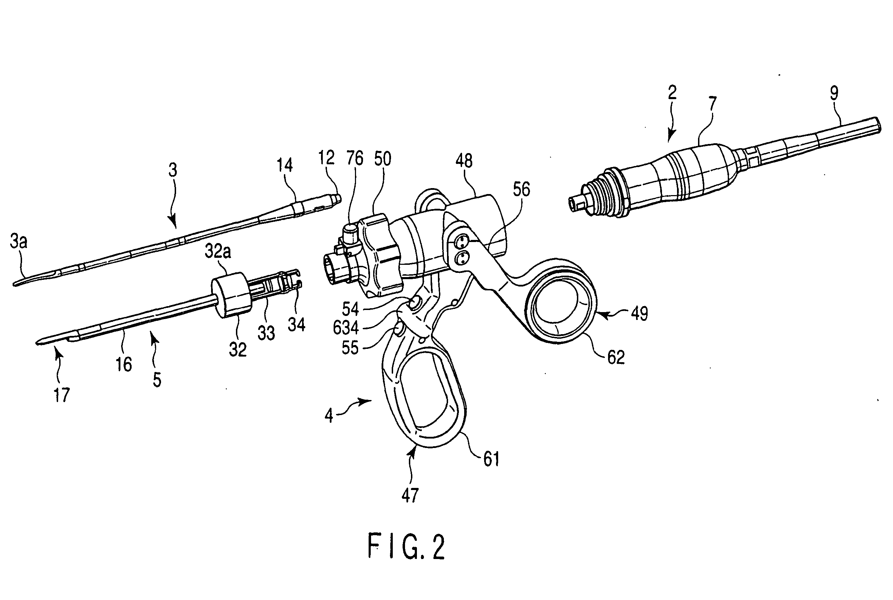

A hand piece 1 includes, as shown in FIG. 2, four nits of a transducer unit 2, a probe unit (probe section) 3, a handle unit (operation section) 4, and a sheath unit (sheath section) 5. These four units are coupled to each other so that they can be disassembled.

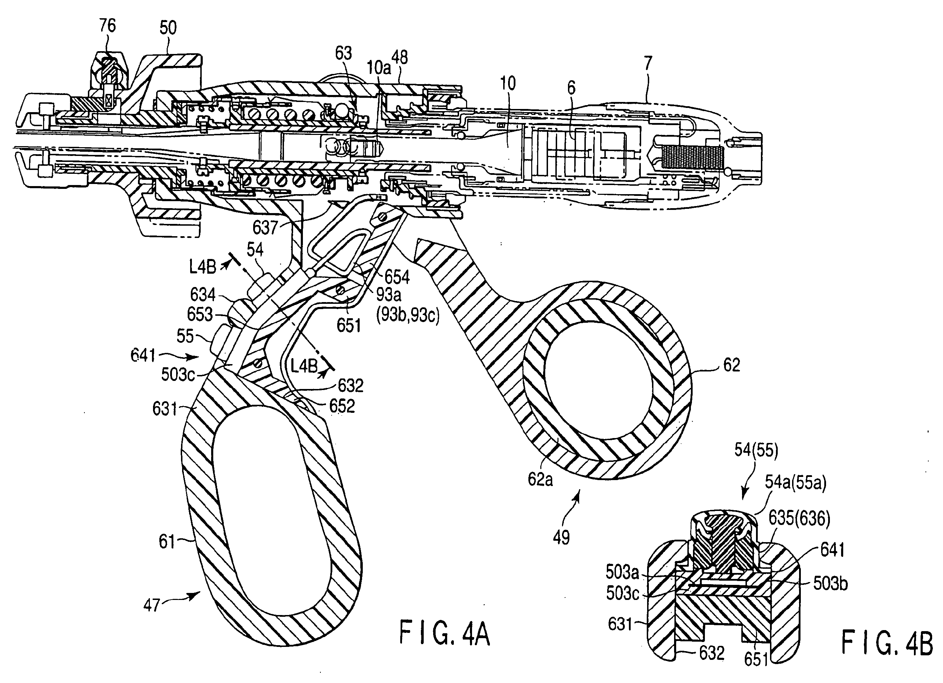

As shown in FIG. 4, an ultrasonic transducer 6 for generating an ultrasonic vibration by means of a piezoelectric element for converting a current into an ultrasonic vibration is incorporated in the transducer unit 2. The outside of the ultrasonic transducer 6 is covered with a cylindrical transducer cover 7. As shown in FIG. 1, a cable 9 for supplying a current for generating an ultrasonic vibration from a power source apparatus main body (not shown) is extended from a rear end of the transducer unit 2.

A proximal end part of a horn 10 for performing amplitude extension of the ultrasonic vibration is coupled to a front end part of the ultrasonic transducer 6. A threaded hole section 10a for attaching a probe is formed in a distal end part of the horn 10.

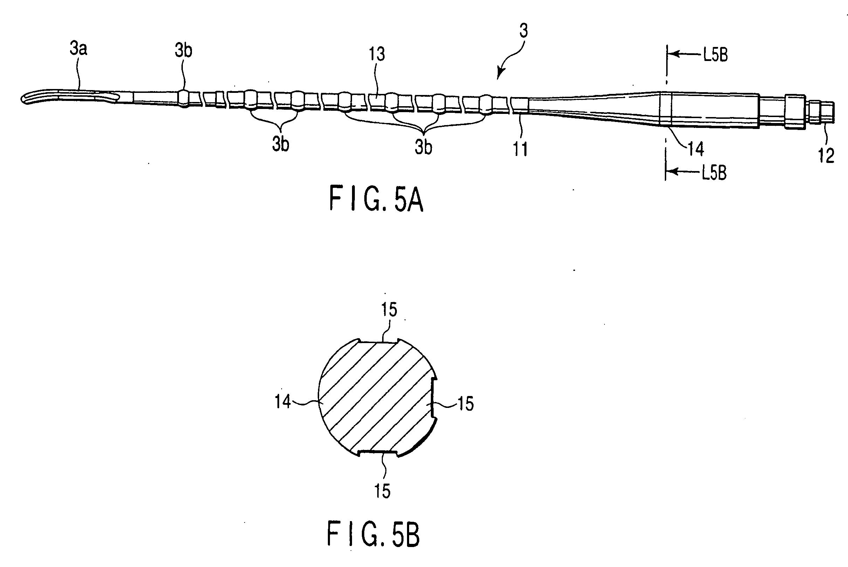

FIG. 5A shows an external appearance of the entire probe unit 3. This probe unit 3 is designed in such a manner that an overall length of the probe unit 3 is an integral multiple of a half wavelength of the ultrasonic vibration. The probe unit 3 includes a vibration transmission member 11 having a distal end part and a proximal end part, and a longitudinal axis, made of metal, and having a stick-like shape. A screw section 12 to be screwed into the threaded hole section 10a of the horn 10 is provided at a proximal end part of the vibration transmission member 11. Further, the screw section 12 is screwed into the threaded hole section 10a of the horn 10 of the transducer unit 2 so as to be attached thereto. As a result of this, the probe unit 3 and the transducer unit 2 are coupled to each other. At this time, in the connected body of the ultrasonic transducer 6 and the probe unit 3, a first high-frequency electric pathway 13 through which a high-frequency current is transmitted is formed.

A probe distal end section 3a is provided at the distal end part of the vibration transmission member 11. The probe distal end section 3a is formed into a curved shape of a substantially J-shape. Further, the probe distal end section 3a constitutes a first electrode section which is one of bipolar electrodes. In the probe unit 3, the cross-sectional area is reduced at several node positions of the vibration on the midway in the axial direction so that an amplitude necessary for the treatment can be obtained at the probe distal end section 3a. Rubber rings 3b formed of an elastic member with a ring-like shape are attached to the probe unit 3 at several node positions of the vibration on the midway in the axial direction of the probe unit 3. Further, these rubber rings 3b prevent interference between the probe unit 3 and the sheath unit 5 from occurring.

A flange section 14 is provided at the closest node position of the vibration to the proximal end side in the axial direction of the probe unit 3. An oddly shaped part having a noncircular shape for preventing a heterogeneous type from being attached is formed on the outer circumferential surface of the flange section 14. This oddly shaped part has a shape obtained by forming, for example, engagement concave sections 15 having a keyway-shape at three positions on the outer circumferential surface of the flange section 14 in the circumferential direction as shown in FIG. 5B.

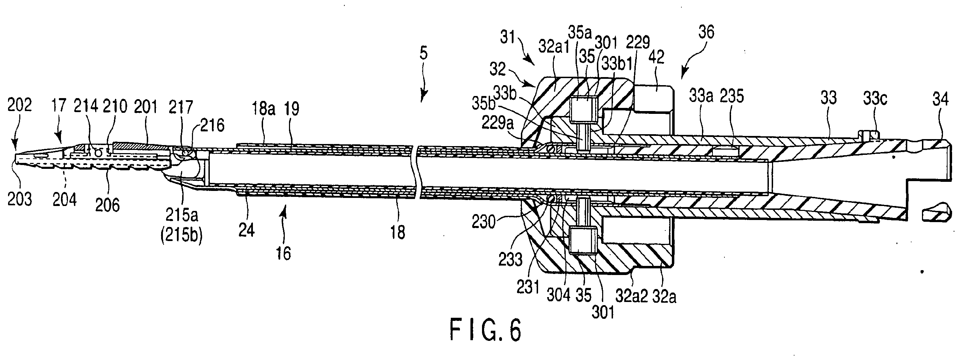

FIG. 6 shows a longitudinal cross-sectional view of the sheath unit 5. The sheath unit 5 includes a sheath main body 16 formed of a cylindrical body, and a jaw 17 arranged at a distal end of the sheath main body 16. The sheath main body 16 includes an outer sheath 18 made of metal which is an external cylinder, and a drive pipe (drive member) 19 made of metal which is an internal cylinder (inner sheath). The drive pipe 19 is inserted into the outer sheath 18 so as to be movable in the axial direction.

An outer circumferential surface of the outer sheath 18 is covered with an outer skin 18a formed of an insulating material such as a resin and the like. An insulating tube 24 formed of an insulating material is arranged on the inner circumferential surface side of the drive pipe 19. A proximal end part of the insulating tube 24 is extended to the proximal end part side of the sheath main body 16. Further, the drive pipe 19 and the probe unit 3 are electrically insulated from each other by the insulating tube 24.

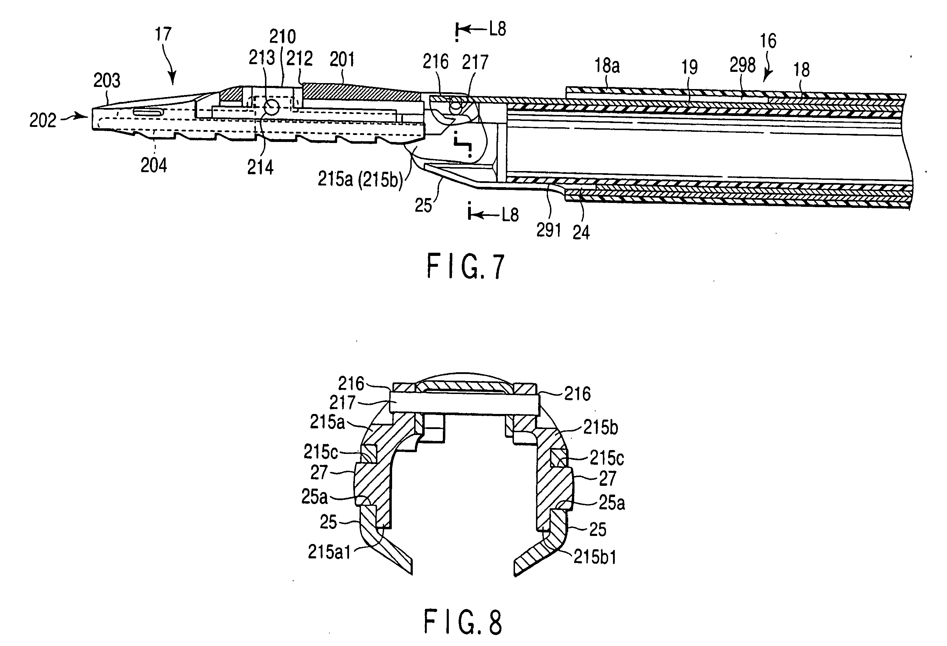

As shown in FIG. 7, a pair of right and left projection pieces 25 (see FIG. 8) are provided to be projected toward the front of the outer sheath 18. As shown in FIG. 8, a circular hole 25a is formed in each of the projection pieces 25. The proximal end part of the jaw 17 is turnably attached to the circular hole 25a of each of the projection pieces 25 through a boss section 27(describes later).

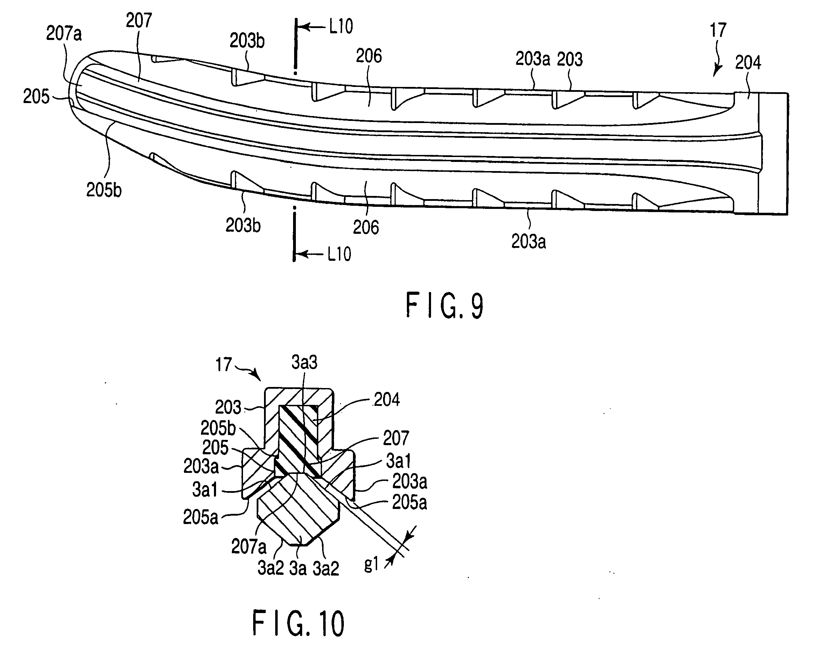

FIG. 9 shows a surface of the jaw 17 at which the jaw is opposed to a probe distal end section 3a. As shown in FIG. 9, the jaw 17 is formed into a curved shape of a substantially J-shape corresponding to the curved shape of the probe distal end section 3a in accordance with the curved shape of the probe distal end section 3a of the probe unit 3. Further, when the probe unit 3 and the sheath unit 5 are coupled to each other, the jaw 17 is arranged at a position at which the jaw 17 is opposed to the probe distal end section 3a of the probe unit 3.

The jaw 17 includes a jaw main body 201 made of metal which is a conductive member, and a grasping member 202 to be attached to the jaw main body 201. The grasping member 202 is constituted of an electrode member 203 for high-frequency treatment, and a pad member 204 (see FIG. 10) for ultrasonic treatment. The electrode member 203 constitutes a second electrode section which is the other of the bipolar electrodes. The pad member 204 is formed of an insulator which is a resin material such as polytetrafluoroethylene and the like.

As shown in FIGS. 9 and 10, a groove section 205 is formed on the undersurface of the electrode member 203 in accordance with the curved shape of the probe distal end section 3a. The pad member 204 is fitted into the groove section 205 in an inserted state.

Inclined surfaces 205a on which the closer a position is to the lower opening surface side, the larger the groove width corresponding to the position becomes are formed on wall surfaces on both sides of the groove section 205 as shown in FIG. 10. Further, as shown in FIG. 9, tooth sections 203b for slip prevention are formed on the lower opening surface side on the wall surfaces 203a on both sides of the groove section 205. These tooth sections 203b constitute a slip prevention section for preventing stuff held between the probe distal end section 3a and the jaw 17 when the jaw 17 and the probe distal end section 3a are engaged with each other from slipping between them. A thickness T of the electrode member 203 is appropriately set in consideration of the rigidity and coagulability.

Further, a notch section 205b is formed at the bottom part of the inclined surfaces 205a of the groove section 205 in the electrode member 203. The notch section 205b is formed in accordance with the curved shape of the probe distal end section 3a. A pressing section 207 of the pad member 204 is arranged in the notch section 205b. As shown in FIG. 10, the pressing section 207 of the pad member 204 is a probe contact member with which the probe distal end section 3a is brought into contact.

An alignment groove 207a is provided at the center of the pressing section 207 of the pad member 204. As shown in FIG. 9, the alignment groove 207a is formed over the entire length of the pad member 204 from the front end part of the pressing section 207 to the rear end part thereof. The probe distal end section 3a is fitted into the alignment groove 207a in an engaged state. Further, in a state where the probe distal end section 3a is fitted into the alignment groove 207a of the pressing section 207 so as to be engaged with the groove 207a, the alignment is performed in a state where the probe distal end section 3a is prevented from being shifted from the electrode member 203 in the lateral direction in FIG. 10. As a result of this, the inclined surfaces 205a of the electrode member 203 and the probe distal end section 3a are prevented from being brought into contact with each other by securing a clearance of a certain distance g1 between the probe distal end section 3a and each of the inclined surfaces 205a of the electrode member 203.

The probe distal end section 3a is formed into a cross-sectional shape shown in FIG. 10. That is, on the top surface side of the probe distal end section 3a, right and left inclined surfaces 3a1 are formed in parallel with the right and left inclined surfaces 205a of the electrode member 203. On the undersurface side of the probe distal end section 3a, right and left inclined surfaces 3a2 are formed in directions opposite to those of the right and left inclined surfaces 3a1. Further, on the top surface side of the probe distal end section 3a, a flat surface section 3a3 parallel with the alignment groove 207a of the pressing section 207 of the pad member 204 is formed between the right and left inclined surfaces 3a1.

Further, the electrode member 203 and the pad member 204 are assembled into an integral body, whereby the grasping member 202 is formed. A projection section 210 for attachment is protrusively provided on the grasping member 202 on the opposite side of the engagement surface 206 at which the jaw 17 is engaged with the probe distal end section 3a. This projection section 210 is screwed onto the jaw main body 201 by means of a fixing screw 214. As a result of this, the grasping member 202 is attached to the jaw main body 201. Here, the electrode member 203 of the grasping member 202 and the jaw main body 201 are electrically connected to each other by means of the fixing screw 214.

The proximal end part of the jaw main body 201 is provided with arm sections 215a and 215b which are formed into two branches. Each of the arm sections 215a and 215b is provided with an extension section 215al or 215b1 extending obliquely below from the center line position of the jaw main body 201. As shown in FIG. 8, the boss section 27 is formed on the outer surface of each of the extension sections 215a1 and 215b1 in a state where the boss section is outwardly protruded from the outer surface. Further, the boss section 27 of each of the extension sections 215a1 and 215b1 is engaged with the circular hole 25a formed in each of the right and left projection pieces 25 of the distal end part of the outer sheath 18 in a state where the boss section 27 is inserted into the circular hole 25a. As a result of this, the jaw main body 201 is turnably attached to the right and left projection pieces 25 of the distal end part of the outer sheath 18 through the boss sections 27.

Further, at a joint part of each of the two arm sections 215a and 215b (upper end part in FIG. 7), a hole 216 into which a connecting pin is to be inserted is formed. A connecting pin 217 for coupling the jaw main body 201 and the drive pipe 19 to each other is fitted in this hole 216. Further, the jaw main body 201 and the drive pipe 19 are electrically connected to each other through the connecting pin 217.

As a result of this, by causing the drive pipe 19 to advance or retreat in the axial direction, the drive force of the drive pipe 19 is transmitted to the jaw 17 through the connecting pin 217. Consequently, the jaw 17 is turnably driven around the fulcrum pin. At this time, when the drive pipe 19 is backwardly pulled, the jaw 17 is turnably driven around the fulcrum pin in a direction in which the jaw 17 is separated from the probe distal end section 3a (toward the opened position). Conversely, when the drive pipe 19 is forwardly pushed, the jaw 17 is turnably driven around the fulcrum pin in a direction in which the jaw 17 is brought closer to the probe distal end section 3a (toward the closed position). The jaw 17 is turnably driven to the closed position, whereby the living tissue is grasped between the jaw 17 and the probe distal end section 3a of the probe unit 3.

The treatment section 1A of the hand piece 1 is constituted of the jaw 17 and the probe distal end section 3a of the probe unit 3. In the treatment section 1A, a plurality of, for example, in this embodiment, two treatment functions (a first treatment function and a second treatment function) can be selected. For example, the first treatment function is set at a function of simultaneously outputting an ultrasonic treatment output and a high-frequency treatment output. The second treatment function is set at a function of outputting only the high-frequency treatment output alone.

Incidentally, the first treatment function and the second treatment function of the treatment section 1A are not limited to the above configurations. For example, a configuration may be employed in which the first treatment function is set at a function of outputting the ultrasonic treatment output in a maximum output state, and the second treatment function is set at a function of outputting the ultrasonic treatment output in an arbitrarily preset output state which is lower than the maximum output state.

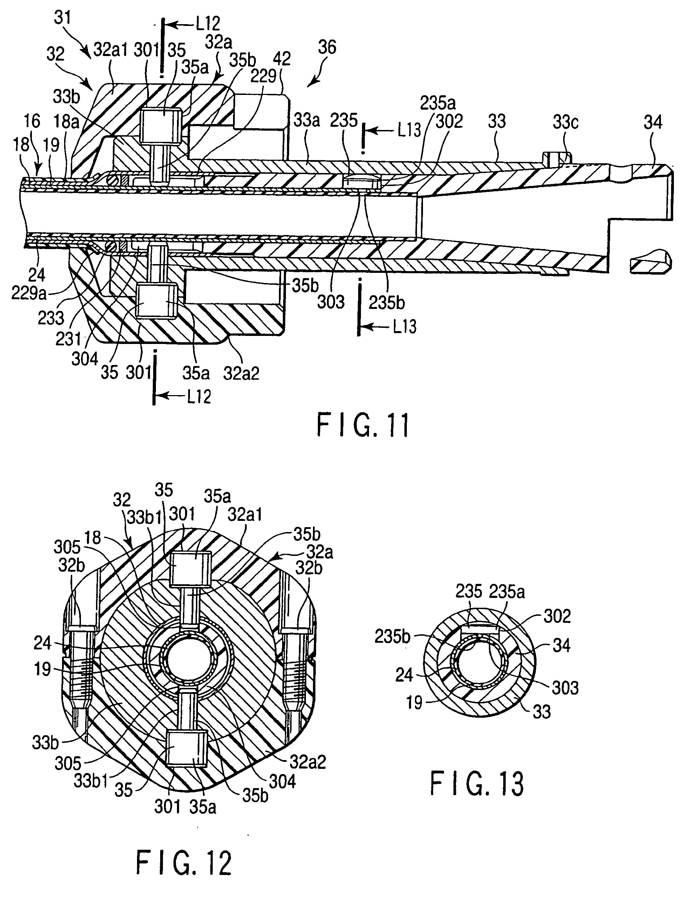

FIG. 11 shows the proximal end part of the sheath main body 16. The proximal end part of the outer sheath 18 is provided with a flare section 229 having an inner diameter larger than that of the other part of the outer sheath 18. The proximal end part of the drive pipe 19 is extended to a position closer to the rear end side than the flare section 229 of the outer sheath 18.

Further, the proximal end part of the sheath main body 16 is provided with an attachment/detachment mechanism section 31 for attaching/detaching the sheath main body 16 to/from the handle unit 4. The attachment/detachment mechanism section 31 is provided with a cylindrical knob member 32 having a large diameter, a guide cylindrical body (first tubular member) 33 constituted of a metallic cylindrical body, and a cylindrical connection tubular body (second tubular member) 34 formed of a resin material.

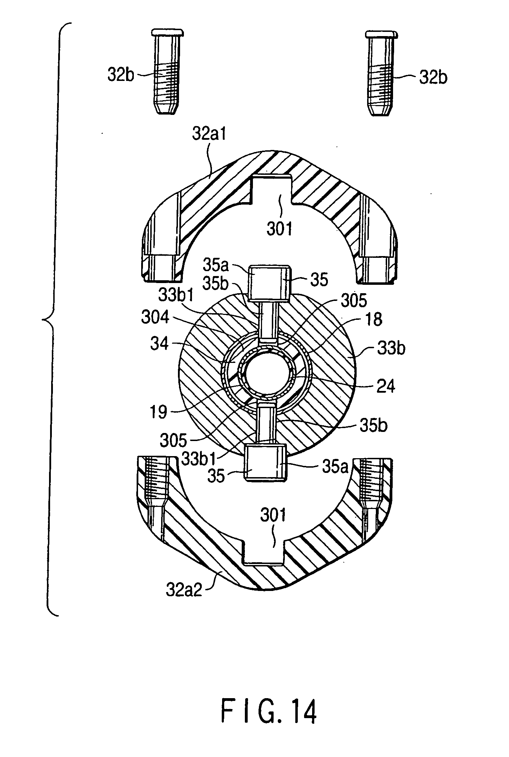

As shown in FIG. 12, the knob member 32 includes a knob main body 32a having a ring-like shape. As shown in FIG. 14, the knob main body 32a includes two C-shaped members 32a1 and 32a2 having a substantially C-shape. These two C-shaped members 32a1 and 32a2 are formed of a resin material, and constitute the knob main body 32a having a ring-like shape in a state where the two C-shaped members 32a1 and 32a2 are coupled to each other at their both ends.

An engagement pit 301 is formed in an inner circumferential surface of each of the two C-shaped members 32a1 and 32a2. A head section 35a of a pin 35 for restraining an internal component from moving is engaged with the engagement pit 301. As a result of this, the position of the pin 35 can be restrained.

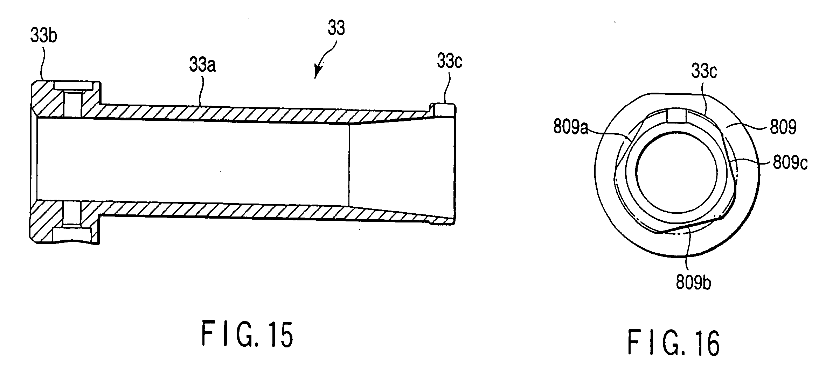

The guide cylindrical body 33 includes a tubular body 33a which is externally fitted on the flare section 229 of the proximal end part of the outer sheath 18, and is backwardly extended. As shown in FIG. 15, the distal end part of the tubular body 33a is provided with a large-diameter section 33b having a larger outer diameter than the other part. The knob member 32 is externally fitted on the large-diameter section 33b. On the outer circumferential surface at the rear end part of the guide cylindrical body 33, an outwardly protruding connection flange section 33c is formed.

In the large-diameter section 33b of the guide cylindrical body 33, two pin-insertion holes 33b1 extending in the radial direction are formed. An axis section 35b of each of the pins 35 is inserted into each of the pin-insertion holes 33b1.

In the flare section 229 of the outer sheath 18, two pin-insertion holes are similarly formed at positions corresponding to the two pin-insertion holes 33b1 of the tubular body 33a. The axis sections 35b of the pins 35 are inwardly protruded through the two pin-insertion holes 33b1 of the tubular body 33a and the two pin-insertion holes of the outer sheath 18. As a result of this, the knob member 32, the guide cylindrical body 33, and the flare section 229 of the outer sheath 18 are assembled into an integral body in a state where the outer sheath 18 is restrained by the pins 35 from moving in the axial direction, and from rotating around the axis.

The connection tubular body 34 is internally fitted into the guide cylindrical body 33 so as to be slidable in the axial direction of the outer sheath 18. A proximal end part of the drive pipe 19 is internally fitted into the inner circumferential surface of the distal end part of the connection tubular body 34.

As shown in FIG. 11, a rotation restraining pin 235 is fixed to the proximal end part of the drive pipe 19. As shown in FIG. 13, the rotation restraining pin 235 includes a large-diameter head section 235a and a small-diameter axis section 235b. In the connection tubular body 34, an engagement hole section 302 to be engaged with the head section 235a of the rotation restraining pin 235 is formed. In the proximal end part of the drive pipe 19, a pin engagement hole 303 to be engaged with the axis section 235b of the rotation restraining pin 235 is formed. Further, the drive pipe 19 and the connection tubular body 34 are coupled to each other by means of the rotation restraining pin 235. At this time, the drive pipe 19 and the connection tubular body 34 are assembled into an integral body in a state where the drive pipe 19 is restrained by the rotation restraining pin 235 from moving in the axial direction, and from rotating around the axis.

The distal end part of the connection tubular body 34 is inserted into the inside of the flare section 229 of the outer sheath 18, and is extended to a position near a step section 229a between the outer sheath 18 and the flare section 229.

Between the flare section 229 and the drive pipe 19, a sealing means 230 for sealing a part between the outer sheath 18 and the drive pipe 19 is provided. The sealing means 230 includes a backup ring 231 and an O-ring 233. The O-ring 233 is provided between the step section 229a of the flare section 229 and the backup ring 231 so as to be movable in the axial direction of the outer sheath 18. Further, the position of the backup ring 231 of the O-ring 233 is restrained on the distal end part of the connection tubular body 34. Further, by utilizing the shape of the step section 229a of the flare section 229, it is possible to cause the step section 229a to double as a backup ring on the front side of the O-ring 233. As a result of this, it is possible to make the number of backup rings 231 of the O-ring 233 only one.

The distal end part of the connection tubular body 34 includes two slits 305 extended in the axial direction of the drive pipe 19. An inner end part of the axis section 35b of each of the pins 35 is inserted in and engaged with each of these slits 305. As a result of this, it is possible to restrain the three parts including the guide cylindrical body 33, the outer sheath 18, and the connection tubular body 34 from moving in the rotational direction with respect to the knob member 32 by the pins 35.

At a rear end part of the knob member 32, an attachment/detachment section 36 for attaching/detaching the knob member 32 to/from the handle unit 4 is arranged. The attachment/detachment section 36 of the knob member 32 is provided with a guide groove (not shown) having an inclined surface-like shape, and an engagement concave section 42. The guide groove is extended in the circumferential direction on the outer circumferential surface of the proximal end part of the knob member 32. Further, the guide groove includes a taper-like inclined surface on which the outer diameter becomes smaller as the diameter becomes closer to the rear end part side of the knob member 32.

The engagement concave section 42 is formed at one end part of the guide groove. The engagement concave section 42 is constituted of a depression section having a diameter smaller than the width of the inclined surface of the guide groove. An engagement lever 43 to be described later on the handle unit 4 side is disengageably engaged with the engagement concave section 42.

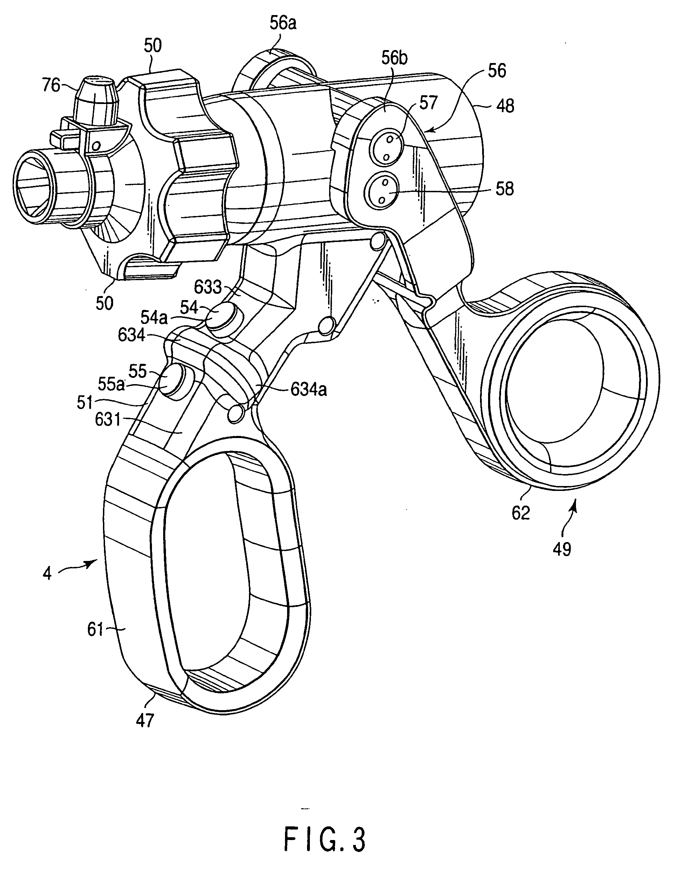

As shown in FIG. 3, the handle unit 4 is mainly includes a fixed handle 47, a retaining cylinder 48, a movable handle 49, and a rotary operation knob 50. The fixed handle 47 includes a plural finger insertion ring section 61 in which, for example, a plurality of fingers of a user other than a thumb is inserted.

As shown in FIG. 4, the fixed handle 47 of this embodiment includes a handle main body 631 in which the retaining cylinder 48 side and the plural finger insertion ring section 61 are formed integral with each other. The handle main body 631 of the fixed handle 47 is provided with a switch holding section 51 between the plural finger insertion ring section 61 and the retaining cylinder 48.

As shown in FIG. 3, the switch holding section 51 includes a switch attachment surface 633 to which a plurality of, for example, in this embodiment, two hand switches (a first switch 54 and a second switch 55) are attached. Each of these first switch 54 and second switch 55 is a switch for selecting a treatment function of the treatment section 1A of the hand piece 1.

In the switch holding section 51, the first switch 54 and the second switch 55 are arranged in the vertical direction. Further, a protuberance section 634 which serves as a partition wall doubling as a finger receiver is formed between the first switch 54 and the second switch 55.

The first switch 54 is arranged on the upper side of the protuberance section 634. The first switch 54 is set as a switch for selecting the first treatment function having the highest frequency in use among the plural treatment functions described previously.

The second switch 55 is arranged on the downside of the protuberance section 634. The second switch 55 is set as a switch for selecting the second treatment function which is another of the plural treatment functions described previously. For example, the first switch 54 is set as a switch button for incision, and the second switch 55 is set as a switch button for coagulation.

The protuberance section 634 is set in such a manner that a height of the protrusion thereof from the switch attachment surface 633 is larger than those of the first switch 54 and the second switch 55. The protuberance section 634 includes an extension section 634a continuously extended from the switch attachment surface 633 of the fixed handle 47 toward both side surfaces.

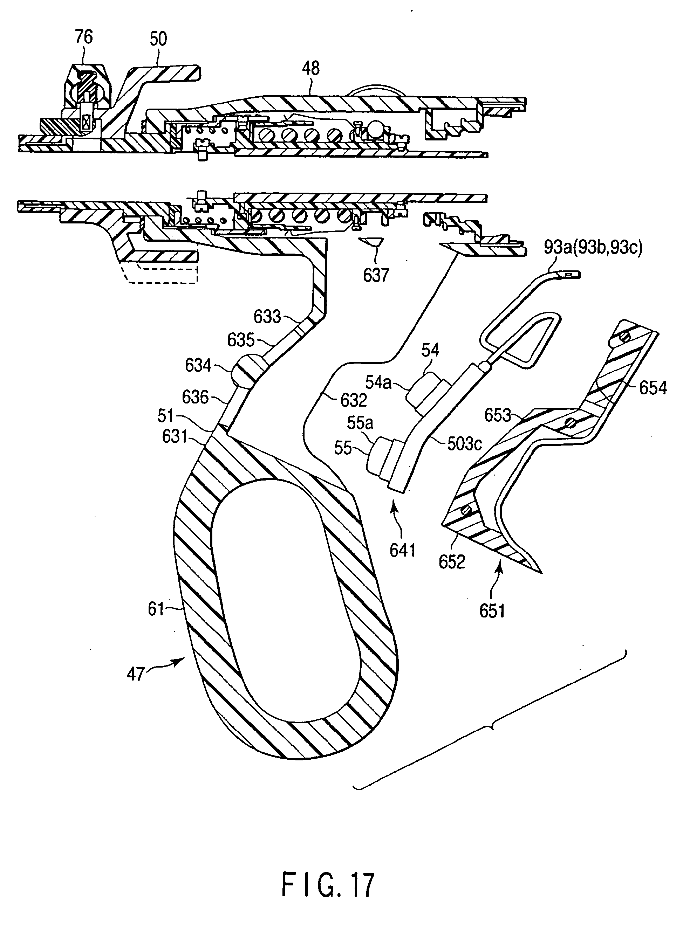

As shown in FIG. 17, the handle main body 631 of the fixed handle 47 includes a concave section 632 on the part of the switch holding section 51 at which the rear part side of the handle main body 631 is opened. The switch attachment surface 633 is formed on the front wall part of the concave section 632.

On the switch attachment surface 633, the protuberance section 634, a first switch button insertion hole 635, and a second switch button insertion hole 636 are formed. The first switch button insertion hole 635 is arranged on the upper side of the protuberance section 634. The second switch button insertion hole 636 is arranged on the downside of the protuberance section 634.

As shown in FIGS. 4A, and 4B, a switch unit 641 and a switch pressing member 651 are fixed to the concave section 632 of the handle main body 631 in a state where the switch unit 641 and the pressing member 651 are inserted therein. The switch unit 641 is formed by integrating the two switches (the first switch 54 and second switch 55) into one unit as shown in FIG. 17.

The switch unit 641 includes a push button 54a for the first switch 54, a push button 55a for the second switch 55, a flexible wiring circuit board 503a for the two switches (the first switch 54 and second switch 55), and a flexible base member 503c in which the wiring circuit board 503a is embedded in two insulating rubber plates (elastic bodies) 503b.

Wiring 93a for the first treatment function one end of which is connected to the first switch 54, wiring 93b for the second treatment function one end of which is connected to the second switch 55, and wiring 93c for the ground one end of which is connected to a common terminal for the ground are connected to the wiring circuit board 503a. These three pieces of the wiring 93a to 93c are contained inside the concave section 632 of the handle main body 631 in a state where the pieces of the wiring are rolled up.

In the switch unit 641, the push button 54a for the first switch 54 is inserted into the first switch button insertion hole 635, and the push button 55a for the second switch 55 is inserted into the second switch button insertion hole 636. In this state, the base member 503c of the switch unit 641 is attached to the concave section 632 of the handle main body 631 in a state where the base member 503c is pressed against the switch attachment surface 633 side from the rear end side by the switch pressing member 651.

As shown in FIG. 17, the switch pressing member 651 includes a guide surface 652, a convex section 653 for pressing the switch unit, and a wiring holding section 654. The guide surface 652 is joined to the concave section 632 of the handle main body 631 along the downside wall surface thereof in FIG. 17.

The convex section 653 for pressing the switch unit presses the base member 503c of the switch unit 641 against the switch attachment surface 633 side. At this time, the base member 503c of the switch unit 641 is tightly pressed against the switch attachment surface 633 side in a state where the base member 503c is bent by the convex section 653 for pressing the switch unit. As a result of this, the base member 503c itself of the switch unit 641 fulfills a function of a gasket, and hence the sealing member or the like around the switch unit 691 can be reduced.

The wiring holding section 654 holds the wiring pieces 93a, 93b, and 93c of the switch unit 641 in the concave section 632 of the handle main body 631.

Further, the handle main body 631 is provided with a boss section 637 between the concave section 632 and the internal space of the retaining cylinder 48 in a protruding manner. This boss section 637 prevents the wiring pieces 93a, 93b, and 93c of the switch unit 641 from entering the internal space side of the retaining cylinder 48 to interfere with the internal operation members in the retaining cylinder 48.

The movable handle 49 includes an arm section 56 having a substantially U-shape at an upper part thereof. At a lower part of the movable handle 49, a finger insertion ring section 62 in which, for example, a thumb of the user is inserted is provided. A ring-shaped finger contact member 62a with a heat-resistant rubber lining is attached to the finger insertion ring section 62.

The U-shaped arm section 56 includes two arms 56a and 56b. The movable handle 49 is attached to the retaining cylinder 48 in a state where the retaining cylinder 48 is inserted between the two arms 56a and 56b.

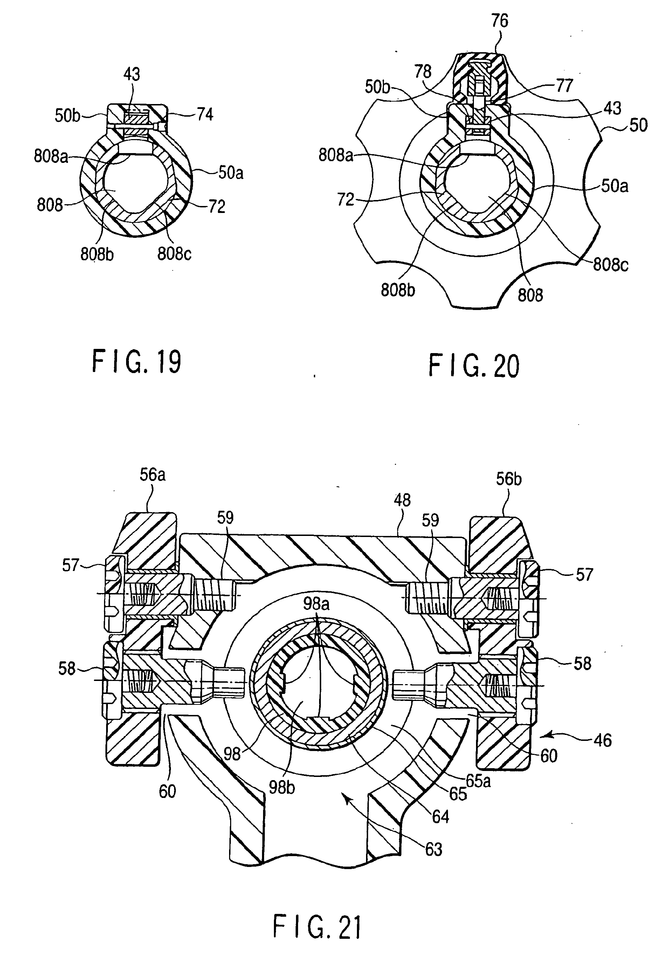

Each of the arms 56a and 56b includes a fulcrum pin 57 and an action pin 58. As shown in FIG. 21, on both sides of the retaining cylinder 48, a pin receiving hole section 59 and a window section 60 are formed. The fulcrum pin 57 of each of the arms 56a and 56b is inserted in the pin receiving hole section 59 of the retaining cylinder 48. As a result of this, an upper part of the movable handle 49 is turnably supported by the retaining cylinder 48 through the fulcrum pins 57. Further, the movable handle 49 is turned around the fulcrum pins 57, and the movable handle 49 is operated to be opened or closed with respect to the fixed handle 47.

Each of the action pins 58 of the movable handle 49 is extended through the window section 60 of the retaining cylinder 48 to the inside of the retaining cylinder 48. A operation force transmission mechanism 63 for transmitting the operation force of the movable handle 49 to the drive pipe 19 of the jaw 17 is provided inside the retaining cylinder 48.

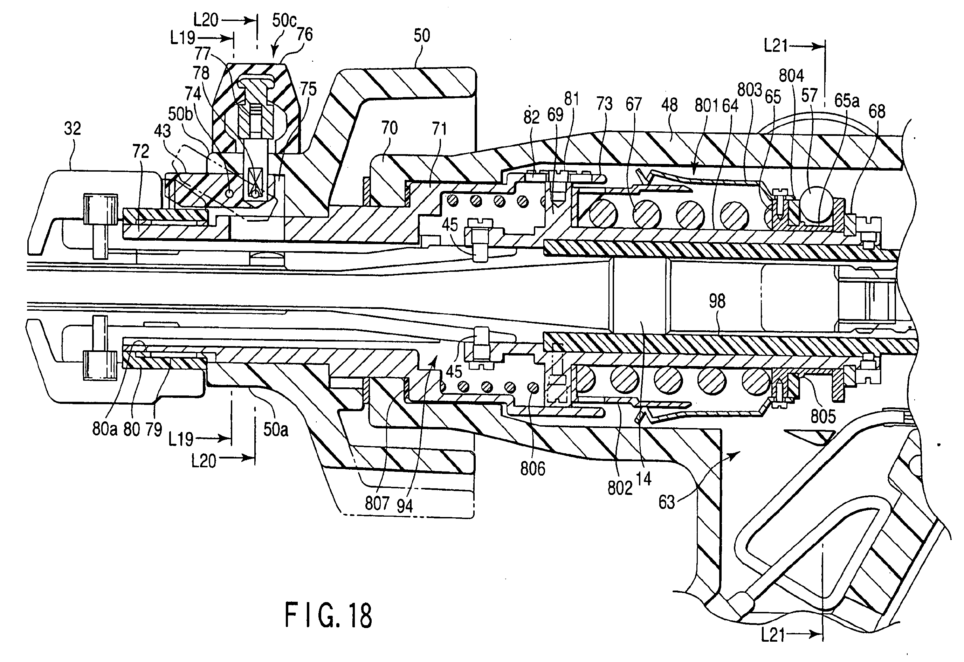

As shown in FIG. 18, the operation force transmission mechanism 63 mainly includes a slider receiving member 64 made of metal and having a cylindrical shape, and a slider member 65. The slider receiving member 64 is arranged concentric with the center line of the retaining cylinder 48, and is extended in the same direction as the insertion direction of the probe unit 3.

A stopper 68, and a spring receiver 69 are arranged on the outer circumferential surface of the slider receiving member 64. The stopper 68 is fixed to the outer circumferential surface of the proximal end part of the slider receiving member 64. The spring receiver 69 is provided on the outer circumferential surface on the distal end part side of the slider receiving member 64 so as to be protruded. Between the stopper 68 and the spring receiver 69, the slider member 65 and a coil spring 67 are arranged. The stopper 68 restrains the movement position of the slider member 65 on the rear end side. The spring receiver 69 is in contact with the front end part of the coil spring 67. The coil spring 67 is arranged between the spring receiver 69 and the slider member 65 with a certain amount of equipped force.

A ring-shaped engagement groove 65a is formed on the outer circumferential surface of the slider member 65 in the circumferential direction. The action pin 58 of the movable handle 49 is engaged with the engagement groove 65a in a state where the action pin 58 is inserted in the engagement groove 65a as shown in FIG. 21. Further, when the movable handle 49 is gripped, and the movable handle 49 is closed with respect to the fixed handle 47, the action pin 58 is rotated around the fulcrum pin 57 concomitantly with the turning operation of the movable handle 49 at this time. The slider member 65 is moved forward in the axial direction (the same direction as the insertion direction of the probe unit 3) concomitantly with the operation of the action pin 58. At this time, the slider receiving member 64 coupled to the slider member 65 through the coil spring 67 is also advanced or retreated together with the slider member 65.

A pair of engagement pins 45 used when the sheath unit 5 and the handle unit 4 side are attached/detached to/from are fixed to the distal end part of the slider receiving member 64. As a result of this, the operation force of the movable handle 49 is transmitted to the connection tubular body 34 of the sheath unit 5 through the pair of engagement pins 45, and the drive pipe 19 of the jaw 17 is moved in the forward direction. As a result, the jaw main body 201 of the jaw 17 is turned around the fulcrum pin.

Further, when the living tissue is grasped between the grasping member 202 of the jaw 17 and the probe distal end section 3a of the probe unit 3 by this operation, the grasping member 202 is turned by a certain amount of angle around the fixing screw 214 as a fulcrum following the bending of the probe distal end section 3a, whereby the force is uniformly applied to the grasping member 202 over the entire length of the grasping member 202. In this state, by outputting the ultrasonic waves, coagulation or incision of the living tissue such as a blood vessel is enabled.

A ring-shaped bearing section 70 is formed at the front end part of the retaining cylinder 48. A cylindrical rotation transmission member 71 made of metal is coupled to the bearing section 70 so as to be rotatable in a direction around the axis. On the rotation transmission member 71, a protrusion section 72 forwardly protruded toward the front of the bearing section 70, and a large-diameter section 73 extended from the bearing section 70 toward the inside of the retaining cylinder 48 are formed.

The rotary operation knob 50 is fixed to the protrusion section 72 in a state where the knob 50 is externally fitted on the protrusion section 72. A fixed ring section 50a having a small diameter is formed at the front end part of the rotary operation knob 50. An outward protrusion section 50b outwardly protruding in the radial direction is formed on a part of the outer circumferential surface of the fixed ring section 50a as shown in FIGS. 19 and 20. The outward protrusion section 50b is provided with an attachment/detachment operation section 50c used for attaching/detaching the handle unit 4 to/from the sheath unit 5.

The attachment/detachment operation section 50c is provided with the engagement lever 43 to be disengageably engaged with the engagement concave section 42 of the knob member 32 of the sheath unit 5. The middle part of the engagement lever 43 is turnably coupled to the outward protrusion section 50b of the rotary operation knob 50 through a pin 74 as shown in FIG. 19. A proximal end part of the engagement lever 43 is extended to the inside of a lever receiving concave section 75 formed in the front of the rotary operation knob 50.

The attachment/detachment operation section 50c of the rotary operation knob 50 is provided with an operation button 76 used to operate the engagement lever 43 in the engagement releasing direction. As shown in FIG. 20, the operation button 76 is provided with a downwardly set operation pin 77 in a protruding manner. The operation pin 77 is extended through a hole in the wall of the outward protrusion section 50b of the rotary operation knob 50 to the inside of the lever receiving concave section 75. A proximal end part of the engagement lever 43 is turnably coupled to the lower end part of the operation pin 77 through a pin 78.

A falling-off prevention ring 80 to prevent the rotary operation knob 50 from falling off is provided at the distal end part of the protrusion section 72 of the rotation transmission member 71. Here, a male thread section 79 is formed at the distal end part of the protrusion section 72. A female thread section 80a to be screw-engaged with the male thread section 79 is formed on the inner circumferential surface of the falling-off prevention ring 80. Further, the female thread section 80a of the falling-off prevention ring 80 is joined to the male thread section 79 of the protrusion section 72 in a screwing manner, whereby the rotary operation knob 50 is fixed to the rotation transmission member 71.

A positioning pin 81 made of metal is attached to the spring receiver 69 of the slider receiving member 64 so as to be outwardly protruded in the radial direction. An engagement hole section 82 having a shape of an elongate hole in which the one pin 81 of the slider receiving member 64 is inserted is formed in the large-diameter section 73 of-the rotation transmission member 71. The engagement hole section 82 is extended in the same direction as the insertion direction of the probe unit 3. As a result of this, when the movable handle 49 is operated, the advancing/retreating operation of the slider receiving member 64 is prevented from being transmitted to the rotation transmission member 71 by moving the pin 81 along the engagement hole section 82.

On the other hand, when the rotary operation knob 50 is operated to be rotated, the rotation operation of the rotation transmission member 71 which rotates together with the rotary operation knob 50 is transmitted to the slider receiving member 64 side through the pin 81. As a result of this, when the rotary operation knob 50 is operated to be rotated, the rotation transmission member 71, pin 81, slider receiving member 64, slider member 65, and coil spring 67 which are in the retaining cylinder 48, and are assembled into a unit are rotationally driven as one body in the direction around the axis together with the rotary operation knob 50.

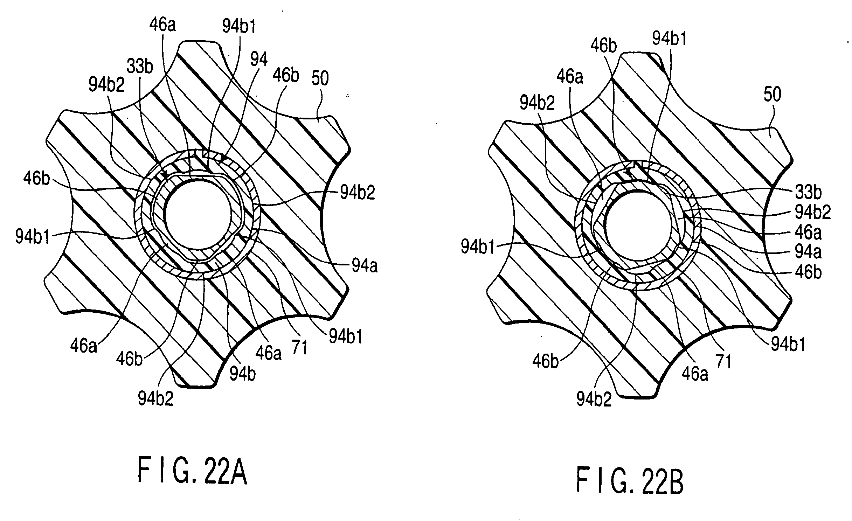

An engagement means 94 which is disengageably engaged with the connection flange section 33c of the sheath unit 5 is provided on the inner circumferential surface of the rotation transmission member 71. FIGS. 22A and 22B show the engagement means 94. This engagement means 94 includes an insertion hole section 94a in which the connection flange section 33c is inserted when the sheath unit 5 and the handle unit 4 are coupled to each other, and a conductive rubber ring (energizing means) 94b arranged in the insertion hole section 94a.

The shape of the inner circumferential surface of the conductive rubber ring 94b is substantially the same as that of an engagement section 46 of the connection flange section 33c. That is, on the inner circumferential surface of the conductive rubber ring 94b, three flat sections 94b1 formed by flattening a plurality of, for example, in this embodiment, three parts of the circular inner circumferential surface, and three corner sections 94b2 arranged at three joint sections between the three flat sections 94b1, and having a larger diameter than the flat sections 94b1 are formed. As a result of this, the conductive rubber ring 94b is formed into a substantially triangular cross-sectional shape. Thus, when the shape of the inner circumferential surface of the conductive rubber ring 94b and the shape of the engagement section 46 of the connection flange section 33c correspond to each other as shown in FIG. 22A, i.e., in a state where the three corner sections 46b of the connection flange section 33c and the three corner sections 94b2 of the conductive rubber ring 94b coincide with each other, the conductive rubber ring 94b is held at a non-compression position in a natural state. On the other hand, when between the handle unit 4 and the sheath unit 5 is relatively rotated in the direction around the center axis of the sheath unit 5, the position of the conductive rubber ring 94b is switched to a compression contract position at which the conductive rubber ring 94b is brought into compression contact with the three corner sections 96b of the connection flange section 33c as shown in FIG. 22B. At this time, the three corner sections 46b of the connection flange section 33c are brought into contact with the three flat sections 94b1 of the conductive rubber ring 94b, whereby the conductive rubber ring 94b is compressed.

In this embodiment, when the sheath unit 5 and the handle unit 4 are coupled to each other, at the insertion operation time when the connection flange section 33c of the sheath unit 5 is inserted straight in the inside of the conductive rubber ring 94b, the conductive rubber ring 94b is held at a non-compression position in the natural state as shown in FIG. 22A. At this time, the engagement lever 43 on the handle unit 4 side is held in a state where the lever 43 runs on the inclined surface of the guide groove of the knob member 32 of the sheath unit 5. Thereafter, by rotating the knob member 32 of the sheath unit 5 in the direction around the axis with respect to the handle unit 4, the engagement lever 43 on the handle unit 4 side is engaged with the engagement concave section 42 at one end part of the guide groove in a state where the engagement lever 43 is inserted in the engagement concave section 42. At this time, the position of the conductive rubber ring 94b is switched to the compression contact position at which the conductive rubber ring 94b is brought into compression contact with the three corner sections 46b of the connection flange section 33c as shown in FIG. 22B. As a result of this, the sheath unit side electric pathway 40 (formed between each of the guide cylindrical body 33, fixing screw 39, joint pipe 38, sheath 18, end cover 25, fulcrum pin, and jaw main body 28) and the handle unit side electric pathway 95 (formed between each of the electric contact point member 96, slider receiving member 64, coil spring 806, and rotation transmission member 71) are electrically connected to each other through the conductive rubber ring 94b. At this time, in the connected body of the sheath unit 5 and the handle unit 4, a second high-frequency electric pathway 97 through which a high-frequency current is transmitted is formed.

The handle unit 4 includes a tubular member 98 formed on the inner circumferential surface of the slider receiving member 64 made of an insulating material. The tubular member 98 is fixed to the inner circumferential surface of the slider receiving member 64. As a result of this, when the probe unit 3 and the handle unit 4 are connected to each other, a first high-frequency electric pathway 13 and the second high-frequency electric pathway 97 are insulated from each other by the tubular member 98.

As shown in FIG. 21, on the inner circumferential surface of the tubular member 98, convex sections 98a are formed at positions corresponding to the three engagement concave sections 15 of the flange section 14 of the probe unit 3. As a result of this, an oddly shaped engagement hole section 98b corresponding to the oddly shaped part of the flange section 14 of the probe unit 3 is formed on the inner circumferential surface of the tubular member 98. When the probe unit 3 and the handle unit 4 are connected to each other, the oddly shaped part of the flange section 14 of the probe unit 3 and the oddly shaped engagement hole section 98b of the tubular member 98 are disengageably engaged with each other. As a result of this, the positions of the probe unit 3 and the tubular member 98 of the handle unit 4 in the rotational direction are restrained. Therefore, when the rotary operation knob 50 is rotated to be operated, the assembled unit inside the retaining cylinder 48, together with the connected body of the probe unit 3 and the transducer unit 2 are driven to be rotated as one body.

Incidentally, the engagement section between the flange section 14 of the probe unit 3 and the tubular member 98 is not limited to the configuration described above. For example, the tubular member 98 may be formed into a cross-sectional shape of a D-shape, and the flange section 14 of the probe unit 3 may be formed into a cross-sectional shape of a D-shape corresponding to the above cross-sectional D-shape.

Further, in the hand piece 1 of this embodiment, a notification mechanism 801 for notifying of a state where the slider member 65 of the operation force transmission mechanism 63 is moved by an amount equal to or larger than the set amount when the movable handle 49 of the handle unit 4 is operated is incorporated.

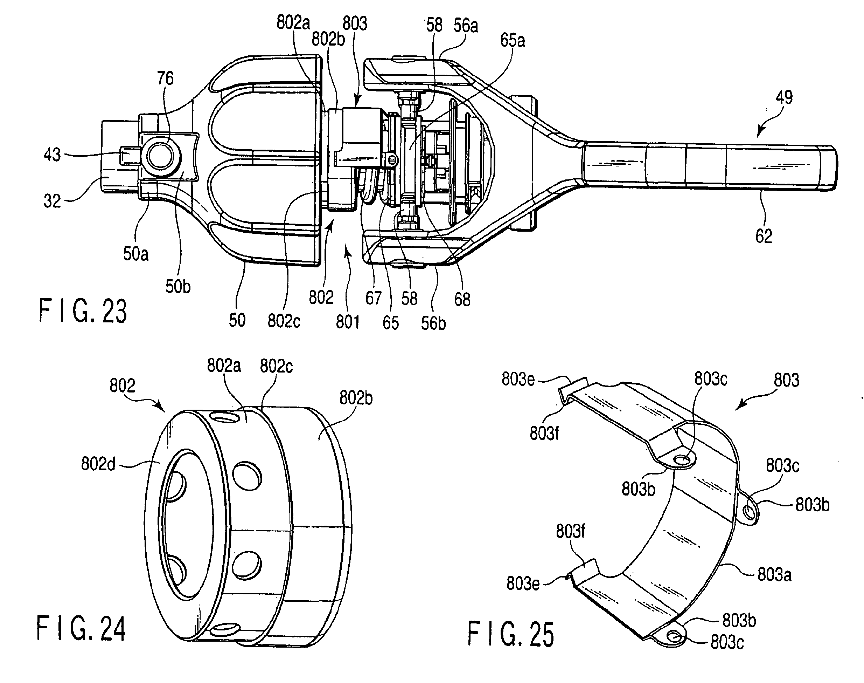

The notification mechanism 801 includes a cylindrical body 802 shown in FIG. 24 and a leaf spring member 803 shown in FIG. 25. The cylindrical body 802 is fixed to the slider receiving member 64 side for guiding the movement of the slider member 65. The leaf spring member 803 is fixed to the slider member 65.

The cylindrical body 802 includes two cylindrical sections 802a and 802b having different diameters. A diameter of the first cylindrical section 802a is formed larger than the diameter of the coil spring 67. The second cylindrical section 802b is formed larger in diameter than the first cylindrical section 802a. Between the first cylindrical section 802a and the second cylindrical section 802b, a step section 802c at which the outer diameter is changed is formed.

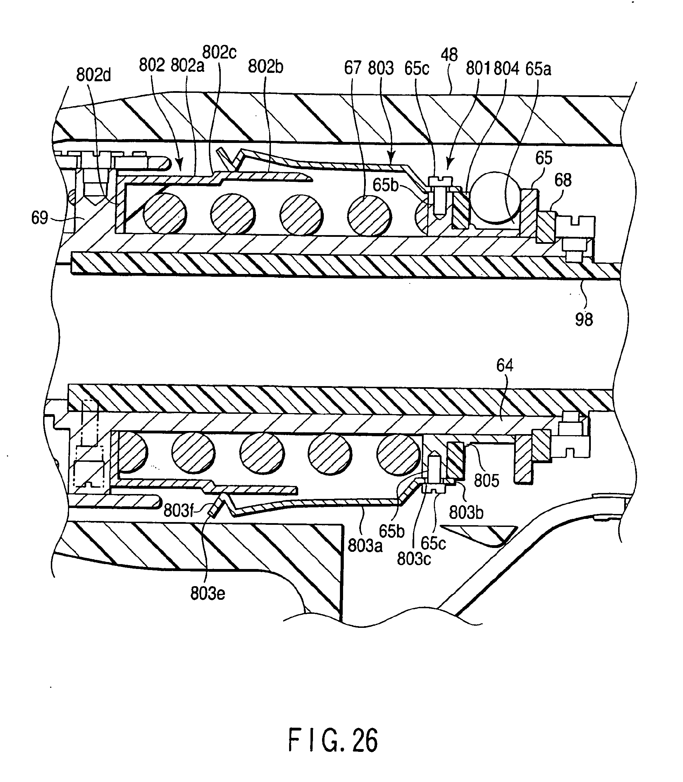

At one end part (end part on the opposite side of the step section 802c) of the first cylindrical section 802a, a joint ring 802d to be joined to the spring receiver 69 of the slider receiving member 64 is provided. As shown in FIG. 26, the cylindrical body 802 is fixed to the slider receiving member 64 side in a state where the joint ring 802d is joined to the spring receiver 69 of the slider receiving member 64.

The leaf spring member 803 includes a leaf spring member main body 803a formed by bending a leaf spring into a substantially semicircular shape. A plurality of, for example, in this embodiment, three bent pieces 803b for fixation are provided at one end part of the leaf spring member main body 803a. A pin insertion hole 803c is formed in each of the bent pieces 803b. Two protrusion pieces 803d that forwardly protrude are provided at the other end part of the leaf spring member main body 803a. A pressure contact section 803e bent inwardly into a substantially V-shape is formed on each of the protrusion pieces 803d.

In the leaf spring member 803, the three bent pieces 803b for fixation are joined to the front end part of the slider member 65. Three screw holes 65b are formed in the front end part of the slider member 65. A screw part of a fixing screw 65c is inserted in the pin insertion hole 803c of each of the bent pieces 803b, and is then screwed into each of the screw holes 65b to be fixed. Here, the pin insertion hole 803c of each of the bent pieces 803b is formed larger in diameter than the screw part of the fixing screw 65c. As a result of this, the leaf spring member 803 is fixed to the front end part of the slider member 65 in a loose-fit state. Further, the pressure contact sections 803e of the leaf spring member main body 803a are set in a state where the sections 803e are in pressure contact with the outer circumferential surface of the cylindrical body 802.

In the notification mechanism 801, when the slider member 65 is operated, the leaf spring member 803 is moved together with the slider member 65, in the same direction as the slider member 65. Further, when the pressure contact sections 803e of the leaf spring member 803 pass the step section 802c between the first cylindrical section 802a and the second cylindrical section 802b, the pressure contact sections 803e fall over the step section 802c. At this time, a knocking sound is generated by the pressure contact sections 803e of the leaf spring member 803 knocking the circumferential wall surface of the first cylindrical section 802a on the lower side of the step section 802c, whereby the state where the slider member 65 of the operation force transmission mechanism 63 has been moved by an amount equal to or larger than a set amount is notified of.

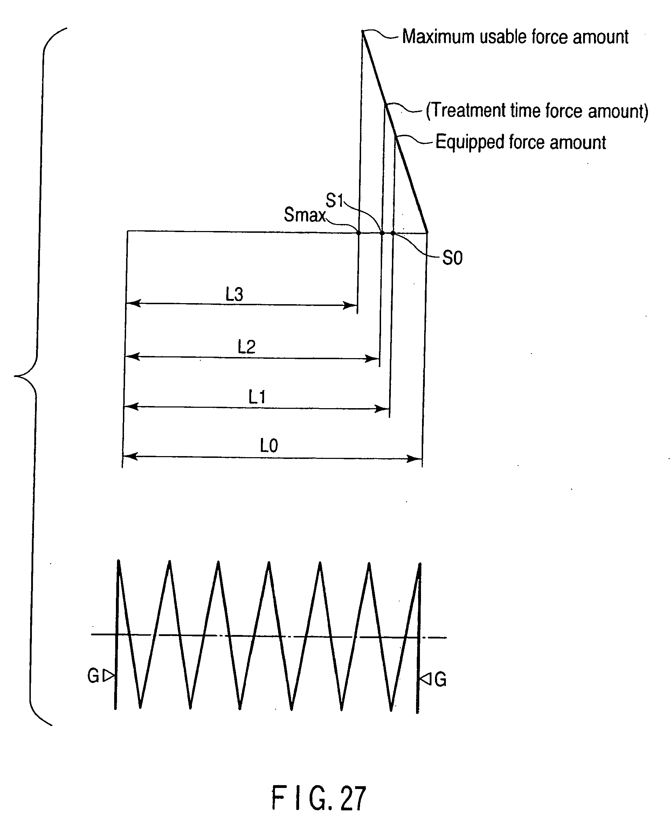

Further, FIG. 27 is an explanatory view for explaining the deformation state of the coil spring 67 at the operation time of the slider member 65. In FIG. 27, a length L0 is a natural length of the coil spring 67, and a length L1 is a set length at the time when the coil spring 67 is set between the spring receiver 69 of the operation force transmission mechanism 63 and the slider member 65 at a certain equipped force amount. In this state, a stroke S of the slider member 65 is S0.

Thereafter, when the movable handle 49 is manipulated in a direction in which the handle 49 is closed, the operation force is transmitted to the slider member 65 according to the operation of the movable handle 49. At this time, the action pin 58 is rotated around the fulcrum pin 57 concomitantly with the turning operation of the movable handle 49. The slider member 65 is moved forward in the axial direction (the same direction as the insertion direction of the probe unit 3) concomitantly with the operation of the action pin 58. At this time, the slider receiving member 64 coupled to the slider member 65 through the coil spring 67 is also advanced or retreated together with the slider member 65.

Further, when the operation force is equal to or larger than a certain amount of the operation force set in advance, the slider receiving member 64 is brought into contact with a stopper, and hence the forward movement is stopped. For this reason, thereafter, only the slider member 65 moves in the forward direction against the spring force of the coil spring 67 concomitantly with the turning operation of the movable handle 49.

Thereafter, at a point of time at which the grasping force for grasping the living tissue between the grasping member 202 of the jaw 17 and the probe distal end section 3a of the probe unit 3 has reached the appropriate set value, the grasping force amount becomes the treatment time force amount. At this time, the stroke S of the slider member 65 is S1, and the length of the coil spring 67 is L2. Incidentally, when the movable handle 49 has reached the maximum turning operation position (maximum usable force amount), the stroke S of the slider member 65 is Smax, and the length of the coil spring 67 is L3.

Further, the notification mechanism 801 of this embodiment is set in such a manner that at a point of time at which the stroke S of the slider member 65 has become S1, and the length of the coil spring 67 has changed to L2, the pressure contact sections 803e of the leaf spring member 803 generate a knocking sound by knocking the circumferential wall surface of the first cylindrical section 802a on the lower side of the step section 802c.

Further, the hand piece 1 of this embodiment is provided with a ring-shaped contact member 804 formed of a slippery resin member, for example, a PTFE (polytetrafluoroethylene) resin material such as Teflon (registered trademark) on the inner wall surface of the engagement groove 65a of the slider member 65. This contact member 804 is arranged on the front side of the action pin 58 of the movable handle 49 inserted in the engagement groove 65a. On the bottom part of the engagement groove 65a, a fixing projection 805 for fixing the contact member 804 to the wall surface on the front side of the engagement groove 65a is provided to be projected.

Further, when the pressing force is applied from the action pin 58 to the wall surface on the front side of the engagement groove 65a, the action pin 58 is prevented from being brought into direct contact with the metal surface of the wall surface on the front side of the engagement groove 65a. As a result of this, in a state where the movable handle 49 is closed with respect to the fixed handle 47, and the living tissue is grasped between the grasping member 202 of the jaw 17 and the probe distal end section 3a of the probe unit 3, the rotation force amount of the rotary operation knob 50 can be made small when the rotary operation knob 50 is operated to be rotated.

Further, the hand piece 1 of this embodiment is provided with a coil spring 806 made of metal or the like and having conductivity between the front surface of the slider member 65 and the rear end surface of the protrusion section 72 of the rotation transmission member 71. By virtue of this coil spring 806, a stable high-frequency current pathway can be secured between the front surface of the slider member 65 and the rotation transmission member 71. Furthermore, the spring force of this coil spring 806 can be made to function as the energizing force for automatically opening the closed movable handle 49.

Further, the hand piece 1 of this embodiment is provided with a bearing section 70 of the retaining cylinder 48 of the fixed handle 47, and a ring-shaped washer 807 formed of a slippery resin member such as Teflon (registered trademark) at the contact surface between the bearing section 70 and the rotation transmission member 71. As a result of this, when rotary operation knob 50 is operated to be rotated, the frictional force acting at the contact surface between the bearing section 70 of the retaining cylinder 48 of the fixed handle 47 and the rotation transmission member 71 can be made small. Thus, the rotation force amount of the rotary operation knob 50 can be made small when the rotary operation knob 50 is operated to be rotated.

Furthermore, as shown in FIGS. 19 and 20, in the hand piece 1 of this embodiment, an oddly shaped hole section 808 having a noncircular shape is formed on the inner circumferential surface of the protrusion section 72 of the rotation transmission member 71. This oddly shaped hole section 808 is constituted of a substantially triangular hole section having three flat surfaces 808a, 808b, and 808c on a circular inner circumferential surface.

Further, as shown in FIG. 16, on the connection flange section 33c of the guide cylindrical body 33 of the sheath unit 5, an oddly shaped engagement section 809 having a noncircular shape corresponding to the oddly shaped hole section 808 is formed. This oddly shaped engagement section 809 is constituted of a substantially triangular flange section having three flat surfaces 809a, 809b, and 809c on a circular outer circumferential surface of the connection flange section 33c.

Further, when the sheath unit 5 and the handle unit 4 are coupled to each other, in a correct case of a combination of units of the same type, the oddly shaped hole section 808 of the rotation transmission member 71 of the handle unit 4 and the oddly shaped engagement section 809 of the guide cylindrical body 33 of the sheath unit 5 can be correctly engaged with each other to be normally coupled to each other.

Next, the function of this embodiment will be described below. The hand piece 1 of the surgical operating apparatus of this embodiment can be disassembled so as to be divided into four units of the transducer unit 2, probe unit 3, handle unit 4, and sheath unit 5 as shown in FIG. 2. Further, when the hand piece 1 is used, the transducer unit 2 and the probe unit 3 are coupled to each other. As a result of this, in the connected body of the transducer unit 2 and the probe unit 3, a first high-frequency electric pathway 13 through which a high-frequency current is transmitted is formed.

Subsequently, the handle unit 4 and the sheath unit 5 are coupled to each other. When the handle unit 4 and the sheath unit 5 are coupled to each other, in a state where the knob member 32 of the sheath unit 5 is grasped, the connection tubular body 34 is inserted into the inside of the rotation transmission member 71 of the handle unit 4. When this sheath unit 5 and the handle unit 4 are coupled to each other, the engagement lever 43 of the handle unit 4 side is held in a state where the engagement lever 43 runs on the inclined surface of the guide groove of the knob member 32 of the sheath unit 5. At this time, as shown in FIG. 22A, the engagement lever 43 is held at a position at which the shape of the inner circumferential surface of the conductive rubber ring 94b and the shape of engagement section 46 of the connection flange section 33c correspond to each other, i.e., in a state where the three corner sections 46b of the connection flange section 33c and the three corner sections 94b2 of the conductive rubber ring 94b coincide with each other. Accordingly, the connection flange section 33c of the sheath unit 5 is inserted straight in the inside of the conductive rubber ring 94b. At this insertion operation time, the conductive rubber ring 94b is held at a non-compression position in the natural state as shown in FIG. 22A. In this state, the sheath unit side electric pathway 40 and the handle unit side electric pathway 95 are not electrically connected to each other.

Subsequently, after this insertion operation is completed, an operation of rotating the knob member 32 of the sheath unit 5 around the axis with respect to the handle unit 4 is performed. By this operation, the engagement lever 43 of handle unit 4 side is engaged with the engagement concave section 42 at one end part of the guide groove in a state where the lever 43 is inserted in the concave section 42. At this time, as shown in FIG. 22B, the position of the conductive rubber ring 94b is switched to the compression contact position at which the rubber ring 94b is brought into compression contact with the three corner sections 46b of the connection flange section 33c. As a result of this, the sheath unit side electric pathway 40 and the handle unit side electric pathway 95 are connected to each other through the conductive rubber ring 94b. As a result of this, in the connected body of the sheath unit 5 and the handle unit 4, a second high-frequency electric pathway 97 through which a high-frequency current is transmitted is formed.

When the sheath unit 5 is operated to be rotated around the axis thereof, the pair of engagement pins 45 of the handle unit 4 side are, at the same time, disengeably engaged with the engagement groove 44a at the termination end of the guide groove 44 of the sheath unit 5. As a result of this, the slider receiving member 64 of the handle unit 4 side and the connection tubular body 34 of the sheath unit 5 side are coupled to each other through the engagement pins 45. As a result of this, it becomes possible for the operation force of the handle unit 4 side at the time when the movable handle 49 is operated to be closed with respect to the fixed handle 47 to be transmitted to the drive pipe 19 of the jaw 17 of the sheath unit 5 side. This state is the state where the sheath unit 5 and the handle unit 4 are coupled to each other.

Thereafter, the connected body of the sheath unit 5 and the handle unit 4, and the connected body of the ultrasonic transducer 6 and the probe unit 3 are assembled into one combined body. At the time of this assembling work, a contact point unit 66 of the handle unit 4 and the front end part of the transducer unit 2 are connected to each other. As a result of this, the second high-frequency electric pathway 97 of the connected body of the sheath unit 5 and the handle unit 4 is connected to the wiring 104 for high-frequency waves transmission inside the cable 9. Further, three wiring lines 105, 106, and 107 inside the cable 9 and a wiring circuit board inside the switch holding section 51 are connected to each other. This state is the completed state of the assembling work of the hand piece 1.

Further, when this hand piece 1 is used, the movable handle 49 is operated to be opened or closed with respect to the fixed handle 47. The drive pipe 19 is moved in the axial direction concomitantly with the operation of the movable handle 49, and the jaw 17 is driven to be opened or closed with respect to the probe distal end section 3a of the probe unit 3 concomitantly with the advancing/retreating operation of the drive pipe 19 in the axial direction. Here, when the movable handle 49 is operated to be closed with respect to the fixed handle 47, the drive pipe 19 is operated to be forwardly pushed concomitantly with the operation of the movable handle 49. The jaw 17 is driven in a direction in which the jaw 17 is made closer to the probe distal end section 3a side of the probe unit 3 (closed position) concomitantly with the pushing operation of the drive pipe 19. When the jaw 17 is operated to be turned to the closed position, the living tissue is grasped between the jaw 17 and the probe distal end section 3a of the probe unit 3.

At this time, in this embodiment, as shown in FIG. 27, at a point of time at which the stroke S of the slider member 65 has changed to S1, and the length of the coil spring 67 has changed to L2, the pressure contact sections 803e of the leaf spring member 803 get over the step section 802c. Thus, the pressure contact sections 803e of the leaf spring member 803 knock the circumferential wall surface of the first cylindrical section 802a on the lower side of the step section 802c, whereby the notification mechanism 801 generates a knocking sound. This makes it possible to notify the user of the appropriate operation amount of the movable handle 49 by a sound.

In this state, either one of the first switch 54 and the second switch 55 of the fixed handle 47 is selectively operated to be depressed. When the first switch 54 is operated to be depressed, a drive current is supplied to the ultrasonic transducer 6 simultaneously with the application of the high-frequency waves, and the ultrasonic transducer 6 is driven. At this time, the ultrasonic vibration from the ultrasonic transducer 6 is transmitted to the probe distal end section 3a through the vibration transmission member 11. As a result of this, it is possible to perform a treatment such as incision, resection, and the like of the living tissue by utilizing the ultrasonic waves together with the application of the high-frequency waves. Incidentally, it is also possible to perform a coagulation treatment of the living tissue by utilizing the ultrasonic waves.

When the second switch 55 is operated to be depressed, each of the first high-frequency electric pathway 13 through which a high-frequency current is transmitted to the probe distal end section 3a of the probe unit 3, and the second high-frequency electric pathway 97 through which a high-frequency current is transmitted to the jaw main body 28 of the sheath unit 5 is turned on. As a result of this, two bipolar electrodes for the high-frequency treatment are constituted by the probe distal end section 3a of the probe unit 3, and the jaw main body 28 of the sheath unit 5. By causing a high-frequency current to flow between the two bipolar electrodes of the probe distal end section 3a of the probe unit 3, and the jaw main body 28 of the sheath unit 5, it is possible to subject the living tissue between the jaw 17 and the probe distal end section 3a of the probe unit 3 to a high-frequency treatment by the bipolar.

Further, when the movable handle 49 is operated to be opened with respect to the fixed handle 47, the drive pipe 19 is operated to be pulled toward the hand side concomitantly with the opening operation of the movable handle 49. The jaw 17 is driven in the direction in which the jaw 17 is separated from the probe distal end section 3a (opened position) concomitantly with the pulling operation of the drive pipe 19.

Further, when the rotary operation knob 50 is operated to be rotated, the rotating operation of the rotation transmission member 71 rotating together with the rotary operation knob 50 is transmitted to the slider receiving member 64 side through the pin 81. As a result of this, when the rotary operation knob 50 is operated to be rotated, the assembled unit of the rotation transmission member 71, pin 81, slider receiving member 64, slider member 65, and coil spring 67 inside the retaining cylinder 48 is driven to be rotated as one body in the direction around the axis together with the rotary operation knob 50. Furthermore, the rotation operation force of the rotary operation knob 50 is transmitted to the vibration transmission member 11 of the probe unit 3 through the tubular member 98 rotating together with the slider receiving member 64 inside the retaining cylinder 48. This allows the assembled unit inside the retaining cylinder 48, together with the connected body of the transducer unit 2 and the probe unit 3 to be rotation-driven as one body in the direction around the axis.

At this time, the knob member 32 of the sheath unit 5, and the guide cylindrical body 33 are rotated together with the rotary operation knob 50. Further, the sheath 18 is rotated together with the guide cylindrical body 33, and the rotation of the guide cylindrical body 33 is transmitted to the connection tubular body 34 and the drive pipe 19 through the rotation restraining pin 235. Accordingly, the jaw 17 of the treatment section 1A, and the probe distal end section 3a are simultaneously driven to be rotated in the direction around the axis together with the rotary operation knob 50.

Thus, the apparatus having the configuration described above produces the following effects. That is, in the hand piece 1 of this embodiment, the notification mechanism 801 can notify the user of the state where the slider member 65 of the operation force transmission mechanism 63 is moved by an amount equal to or larger than the set amount when the movable handle 49 of the handle unit 4 is operated by a sound. At this time, in the notification mechanism 801, in FIG. 27, at a point of time at which the stroke S of the slider member 65 has changed to S1, and the length of the coil spring 67 has changed to L2, the pressure contact sections 803e of the leaf spring member 803 get over the step section 802c. Thus, the pressure contact sections 803e of the leaf spring member 803 knock the circumferential wall surface of the first cylindrical section 802a on the lower side of the step section 802c, whereby the notification mechanism 801 generates a knocking sound. This makes it possible to notify the user of the appropriate operation amount of the movable handle 49 by a sound. As a result of this, it is possible to prevent the operation force of the movable handle 49 from being varied depending on the user, prevent the treating capability at the time when the incision, resection, or coagulation of the living tissue is performed by the ultrasonic vibration from being varied, and perform a stable operation.

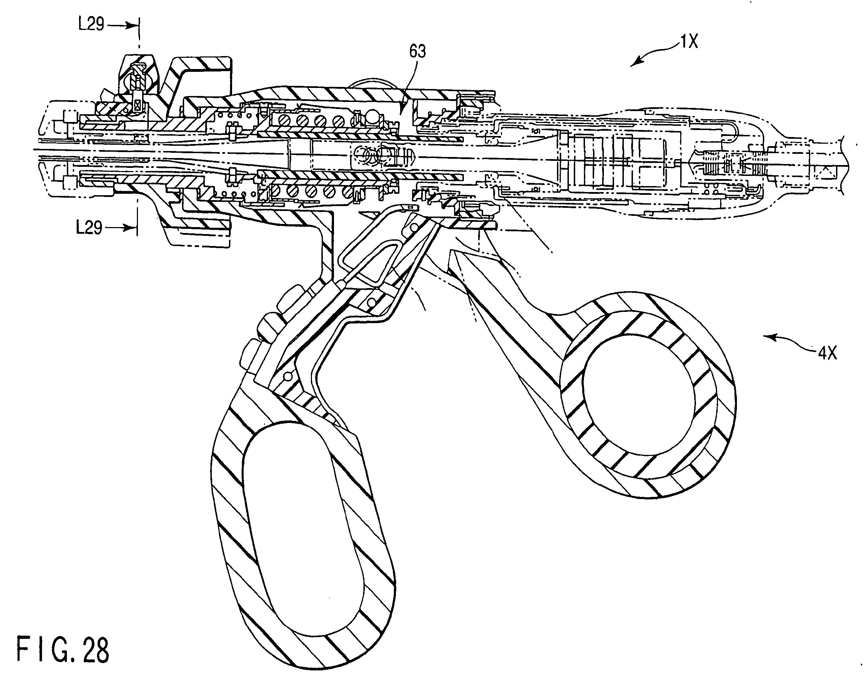

FIG. 28 shows an internal structure of a handle unit of a hand piece of a second type different from the hand piece 1 of the surgical operating apparatus of the first embodiment, for example, a handle unit 4X of a hand piece 1X having a treating function of ultrasonic treatment alone.

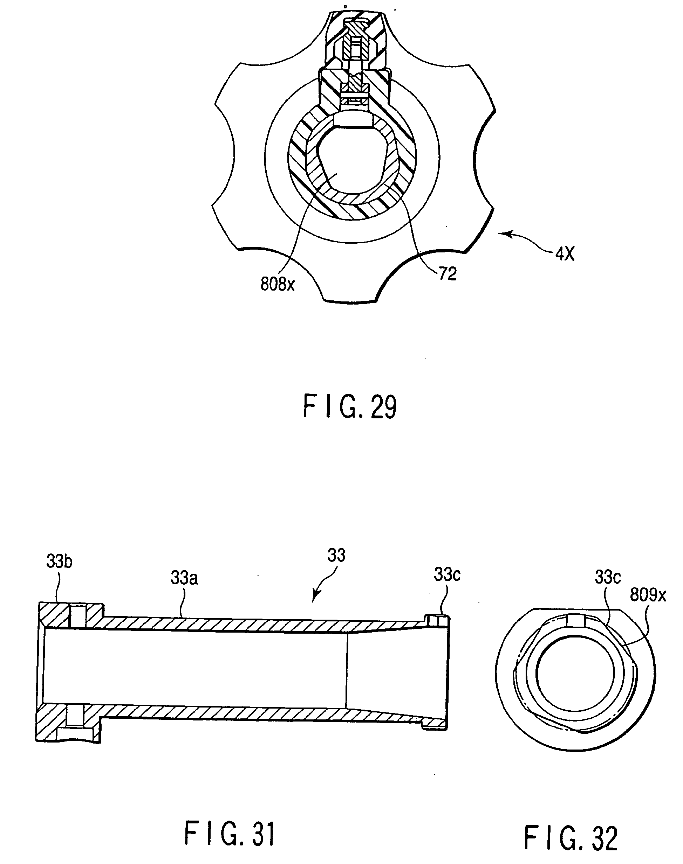

In the hand piece 1X, a noncircular oddly shaped hole section 808X is formed on the inner circumferential surface of a protrusion section 72 of a rotation transmission member 71 as shown in FIG. 29. This oddly shaped hole section 808X is constituted of a hole section an inner circumferential surface of which is formed into a substantially square shape.

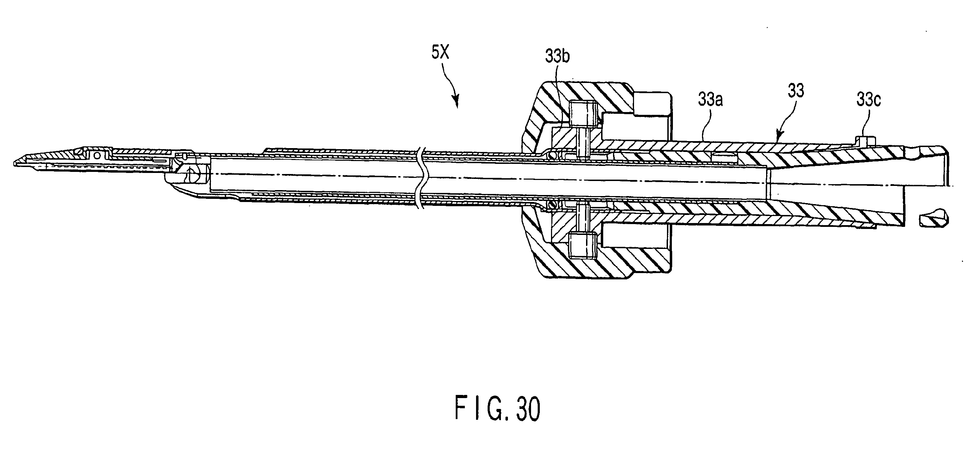

Further, FIG. 30 shows an internal structure of the sheath unit 5X of the second type. In the sheath unit 5X of the second type, a noncircular oddly shaped engagement section 809X corresponding to the oddly shaped hole,section 808X is formed on a connection flange section 33c of a guide cylindrical body 33 as shown in FIGS. 31 and 32. In this oddly shaped engagement section 809X, the outer circumferential surface of the connection flange section 33c is constituted of a substantially square flange section.

Further, when the sheath unit 5X and the handle unit 4X are coupled to each other, in the case of a correct combination of units of the same type, the oddly shaped hole section 808X of the rotation transmission member 71 of the handle unit 4X, and the oddly shaped engagement section 809X of the guide cylindrical body 33 of the sheath unit 5X can be correctly engaged with each other, and normally coupled to each other.

Further, when, for example, the sheath unit 5 of the hand piece 1 of the surgical operating apparatus according to the first embodiment, and the handle unit 4X of the second type are combined with each other, the substantially square oddly shaped hole section 808X of the rotation transmission member 71 of the handle unit 4X, and the oddly shaped engagement section 809 of the substantially triangular guide cylindrical body 33 of the sheath unit 5 according to the first embodiment are made to be engaged with each other. In this case, the shapes of both the units are different from each other, and hence they cannot be correctly engaged with each other. Thus, they cannot be normally coupled to each other, and hence an erroneous combination of the sheath unit 5 and the handle unit 4 can be prevented from occurring.

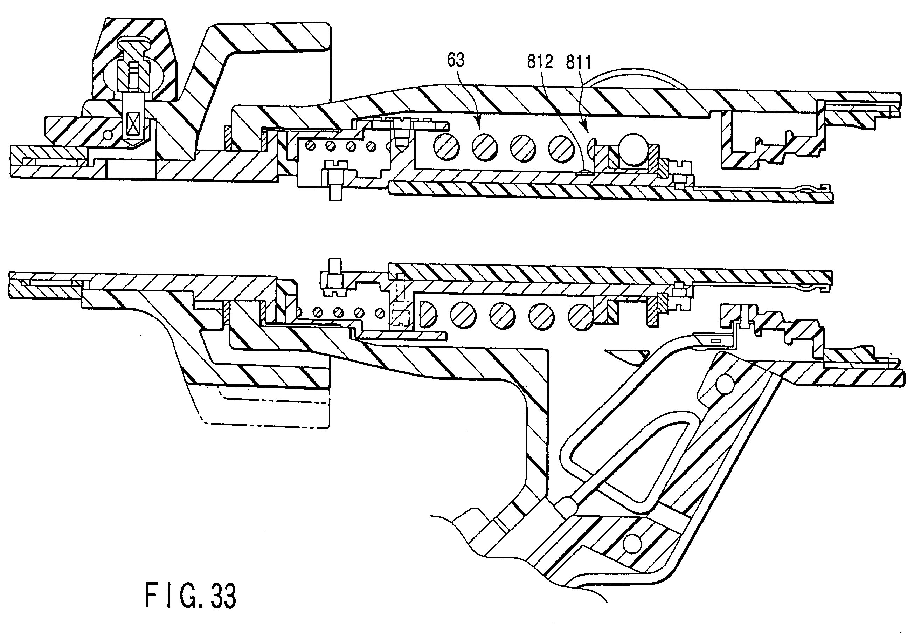

Further, FIGS. 33 to 36 show a second embodiment of the present invention. In this embodiment, a notification mechanism 811 different from the notification mechanism 801 of the first embodiment (FIGS. 1 to 27) is provided.

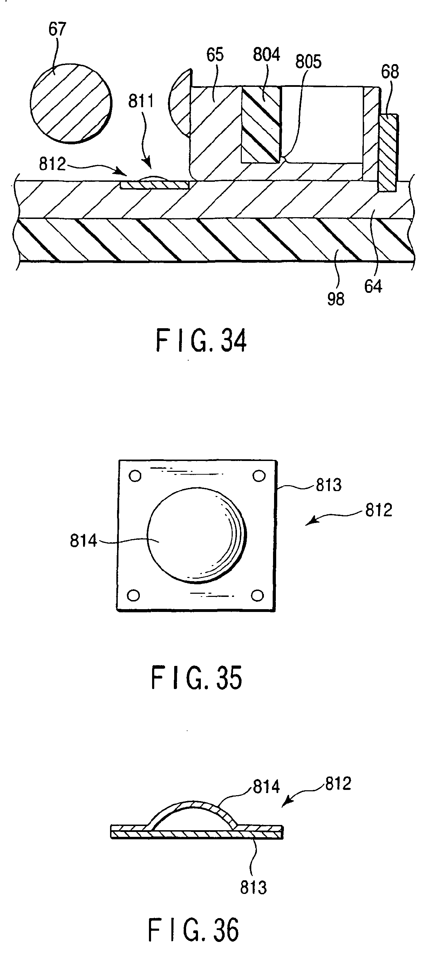

FIG. 33 shows the attached state of the notification mechanism 811 of this embodiment. In the notification mechanism 811, a sound generation piece 812 is fixed to an outer circumferential surface of a slider receiving member 64. This sound generation piece 812 includes, as shown in FIG. 35, a rectangular base plate 813, and a substantially hemispherical leaf spring member 814 fixed to the base plate 813.

Further, at the time of the operation of the slider member 65, when the slider member 65 gets over the sound generation piece 812 on the outer circumferential surface of the slider receiving member 64, a knocking sound is generated by the elastic deformation of the leaf spring member 814, whereby a state where the slider member 65 of a operation force transmission mechanism 63 has been moved by a set amount or more is notified of.

Thus, in this embodiment having the configuration described above too, the appropriate operation amount of the movable handle 49 can be notified to the user by a sound. As a result of this, it is possible to prevent the operation force of the movable handle from being varied depending on the user, prevent the treating capability at the time when the incision, resection, or coagulation of the living tissue is performed by the ultrasonic vibration from being varied, and perform a stable operation.

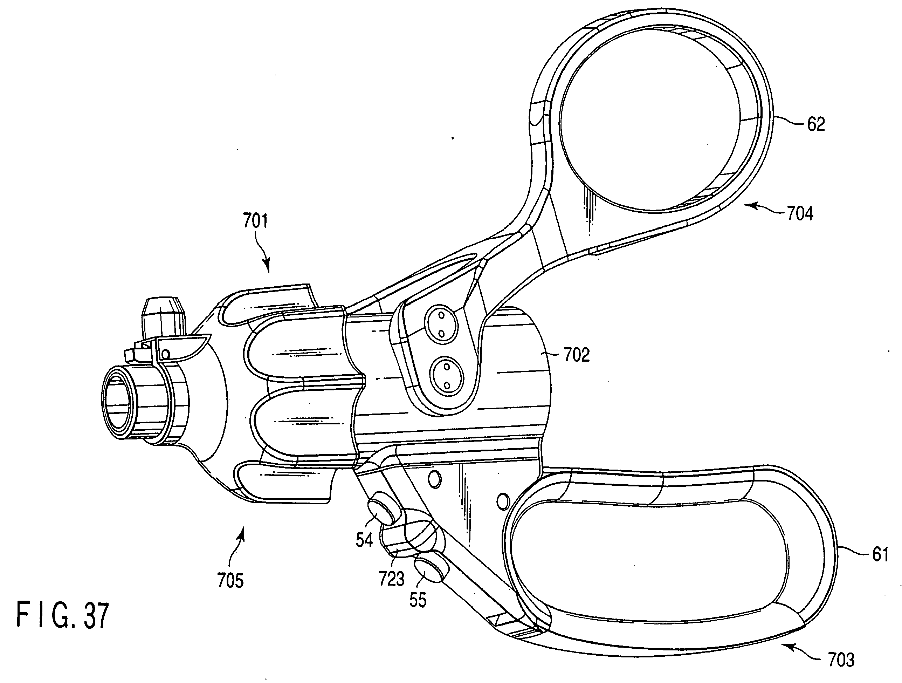

Further, FIGS. 37 to 40 show a third embodiment of the present invention. In this embodiment, a notification mechanism 801 identical with that of the first embodiment (FIGS. 1 to 27) is incorporated in a hand piece 821 having a different configuration from that of the first embodiment.

In a hand piece 701 of this embodiment, a fixed handle 703 is fixed to one side part of a retaining cylinder 702. Further, a movable handle 704 is arranged at the other side part of the retaining cylinder 702, i.e., a side part opposite to the fixation part side of the fixed handle 703. Incidentally, a reference symbol 705 denotes a rotary operation knob.

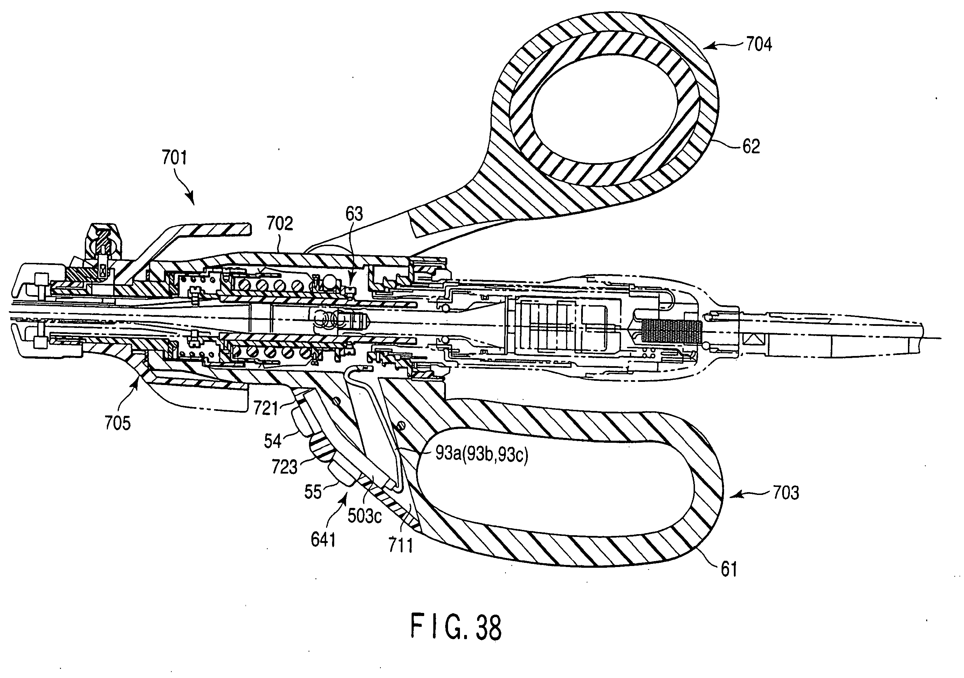

In this embodiment, as shown in FIG. 38, the fixed handle includes a handle main body 706 formed integral with the retaining cylinder 702.

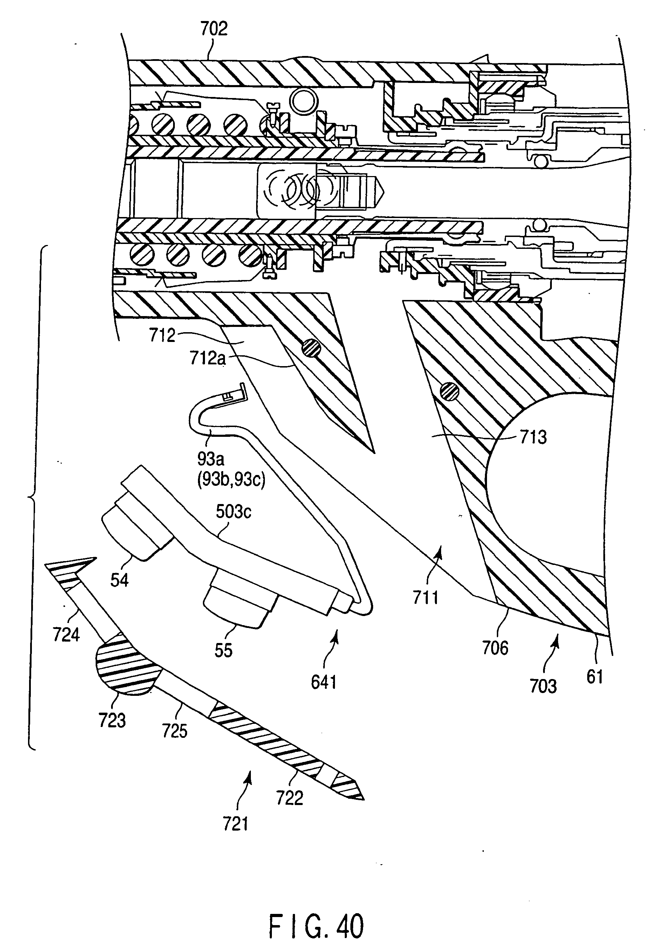

As shown in FIG. 40, the handle main body 706 includes a switch attachment concave section 711 between a plural finger insertion ring section 61 and the retaining cylinder 702. The concave section 711 is opened at the front side of the handle main body 706. The concave section 711 includes a switch unit pressing section 712, and a wiring insertion section 713. A switch attachment surface 712a having a curved shape is formed on the inner bottom part of the switch unit pressing section 712.

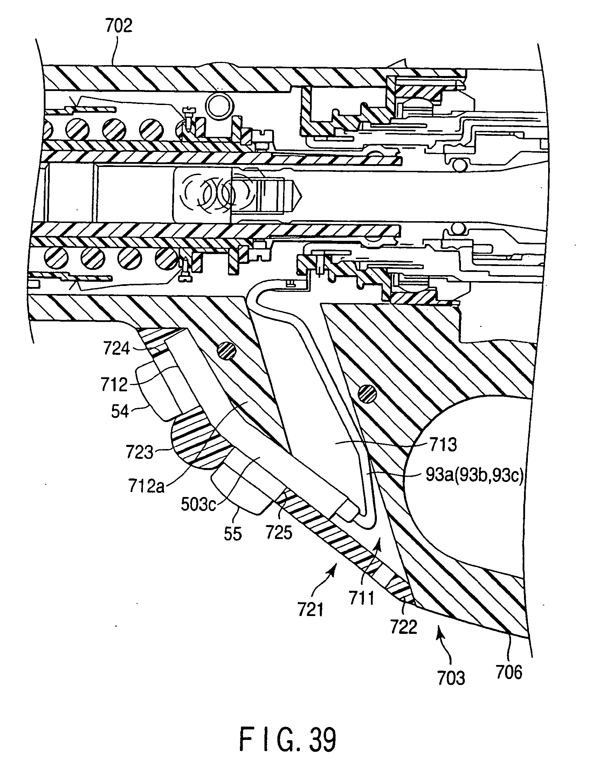

As shown in FIGS. 38 and 39, in the wiring insertion section 713, wiring pieces 93a, 93b, and 93c of the switch unit 641 are inserted. The base member 503c of the switch unit 641, and the plate-like switch pressing member 721 are fixed to the switch unit pressing section 712 in a state where the base member 503c and the switch pressing member 721 are inserted in the switch unit pressing section 712.

In the switch pressing member 721, a protuberance section 723 which serves as a partition wall doubling as a finger receiver is formed on the plate-like main body 722. A first switch button insertion hole 724 is formed on the upper side of the protuberance section 723. A second switch button insertion hole 725 is formed on the lower side of the protuberance section 723.

Here, in the switch unit 641, the push button 54a for the first switch 54 is inserted into the first switch button insertion hole 724, and the push button 55a for the second switch 55 is inserted into the second switch button insertion hole 725. In this state, the base member 503c of the switch unit 641 is inserted into the switch unit pressing section 712 from the front side. Further, the base member 503c is attached to the handle main body 706 in a state where the base member 503c is pressed against the switch attachment surface 712a side of the switch unit pressing section 712 by the switch pressing member 721, and is bent.

Further, an operation force transmission mechanism 63 for transmitting the operation force of the movable handle 704 to the drive pipe 19 of the jaw 17, and having the same configuration as that of the first embodiment is provided inside the retaining cylinder 702 of the fixed handle 703. A notification mechanism 801 identical with that of the first embodiment is incorporated in the operation force transmission mechanism 63.

Thus, in the hand piece 1 of this embodiment having the configuration described above, when the work for attaching the switch unit 641 to the fixed handle 703 is performed, the wiring pieces 93a, 93b, and 93c of the switch unit 641 are inserted in the wiring insertion section 713. After that, the base member 503c of the switch unit 641, and the switch pressing member 721 are inserted in sequence in the switch unit pressing section 712. Further, the configuration is made such that the switch unit 641 is pressed against the switch attachment surface 712a side by the switch pressing member 721. Thus, the work for attaching the switch unit 641 to the fixed handle 703 can be easily performed.

Furthermore, the base member 503c of the switch unit 641 is pressed in a state where the base member 503c is pressed against the switch attachment surface 712a side by the switch pressing member 721. As a result of this, the base member 503c itself of the switch unit 641 fulfills a function of a gasket, and hence the sealing member or the like around the switch unit 641 can be reduced. Thus, the work for attaching the switch unit 641 can be performed further easier.

Further, the notification mechanism 801 identical with that of the first embodiment is incorporated in the operation force transmission mechanism 63 inside the retaining cylinder 702 of the fixed handle 703. Thus, in this embodiment too, it possible to notify the user of the appropriate operation amount of the movable handle 704 by the sound of the notification mechanism 801. As a result of this, it is possible to prevent the operation force of the movable handle 704 from being varied depending on the user, prevent the treating capability at the time when the incision, resection, or coagulation of the living tissue is performed by the ultrasonic vibration from being varied, and perform a stable operation.

Incidentally, the present invention is not limited to the embodiments described above. Needless to say, the present invention can be variously modified to be implemented within the scope of the claims.

## Claims

1. A surgical operating apparatus comprising:
a sheath (18) provided with a distal end part and a proximal end part;
an apparatus main body to be coupled to the proximal end part of the sheath (18);
a probe (11) which is provided with a distal end part and a proximal end part, is inserted into the sheath (18), and transmits ultrasonic vibration from the proximal end part side to the distal end part side;
a jaw (17) which is turnably supported at the distal end part of the sheath (18), and can be operated to be opened or closed between a closed position at which the jaw (17) is engaged with the distal end part (3a) of the probe (11), and an opened position at which the jaw (17) is separated from the distal end part (3a) of the probe (11);
a handle (49) which is provided on the apparatus main body, and is adapted to operate the opening/closing operation of the jaw (17) according to an operation force of the handle (49);
a slider section (65) which is provided in the apparatus main body, and can be advanced/retreated to be moved in a central axis direction of the probe (11) between a first movement position corresponding to the opened position of the jaw (17) and a second movement position corresponding to the closed position of the jaw (17) in accordance with the operation of the handle (49);
a slider receiving member (64) which is provided in the apparatus main body, and is adapted to guide the movement of the slider section (65);
a spring receiver (69) arranged on an outer circumferential surface of the slider receiving member (64);
a coil spring (67) being arranged between the slider section (65) and the spring receiver (69); and
a stopper configured to be brought into contact with the slider receiving member (64);
wherein, when the slider section (65) is moved from the first movement position to the second position in accordance with the operation of the handle (49), the slider receiving member (64) is also advanced together with the slider section (65);
when the operation force of the handle (49) is larger than a predetermined amount, the slider receiving member (64) is brought into contact with the stopper, and, thereafter, only the slider section (65) moves in a distal direction against a spring force of the coil spring (67) according to operation of the handle (49), and thereafter, when a length of the coil spring (67) is changed to a length (L2) corresponding to a movement of the slider section (65) of a set amount (S1), wherein the set amount (S1) corresponds to a predetermined grasping force for grasping living tissue by the jaw (17), a notification mechanism (801, 811) notifies of a state where the slider section (65) has moved by an amount equal to or larger than the set amount (S1) on the way thereof from the first movement position to the second movement position.

2. The surgical operating apparatus according to claim 1, wherein the notification mechanism (801, 811) includes a notification section which is adapted to perform notification by means of at least one of a sound and a feeling of a click.

3. The surgical operating apparatus according to claim 2, wherein the notification section includes:
a cylindrical body (802) that is fixed to the side of the slider receiving member (64), and is provided with a step section (802c) on an outer circumferential surface thereof at which a diameter thereof is changed at a position corresponding to the set amount (S1) in a movement direction of the slider section (65), and
a leaf spring member (803) that is provided with first and second end parts (803b, 803d), wherein a first end part (803b) is fixed to the slider section (65), and the second end part (803d) is held on the outer circumferential surface of the cylindrical body (802) in a state where the second end part (803d) is in sliding contact with the outer circumferential surface of the cylindrical body (802), and
at the time of the movement of the slider section (65), when the leaf spring member (803) passes the step section (802c), the second end part (803d) of the leaf spring member (803) is adapted to fall over the step section (802c), and is adapted to generate a knocking sound by knocking the circumferential wall (802a) surface on the lower side of the step section (802c).

4. The surgical operating apparatus according to claim 2, wherein the notification section includes a sound generation member (812) which is provided on the slider receiving member (64), and is adapted to generate the sound by being brought into contact with the slider section (65) in the state where the slider section (65) has moved by the amount equal to or larger than the set amount (S1) on the way thereof from the first movement position to the second movement position.

5. The surgical operating apparatus according to claim 1, wherein the set amount (S1) is set at a position corresponding to a operation amount of the handle (49), the operation amount enabling the appropriate ultrasonic treating capability to be exerted when the living tissue is grasped between the jaw (17) and the distal end part of the probe (11).

6. The surgical operating apparatus according to claim 5, wherein the ultrasonic treating capability is at least one of coagulation and incision.

## Patentansprüche

1. Chirurgisches Operationsgerät, das umfasst:
eine Hülse (18), die mit einem distalen Endteil und einem proximalen Endteil versehen ist;
einen mit dem proximalen Endteil der Hülse (18) zu koppelnden Gerätehauptkörper;
eine Sonde (11), die mit einem distalen Endteil und einem proximalen Endteil versehen ist, in die Hülse (18) eingeführt ist und eine Ultraschallschwingung von der proximalen Endteilseite zu der distalen Endteilseite überträgt;
eine Klaue (17), die drehbar an dem distalen Endteil der Hülse (18) gelagert ist und betätigt werden kann, um zwischen einer geschlossenen Position, in der sich die Klaue (17) in Eingriff mit dem distalen Endteil (3a) der Sonde (11) befindet, und einer geöffneten Position, in der die Klaue (17) von dem distalen Endteil (3a) der Sonde (11) getrennt ist, geöffnet oder geschlossen zu werden;
einen Griff (49), der an dem Gerätehauptkörper vorgesehen ist und dazu eingerichtet ist, die Öffnung/Schließ-Betätigung der Klaue (17) gemäß einer Betätigungskraft des Griffs (49) zu betätigen;
einen Schieberabschnitt (65), der in dem Gerätehauptkörper vorgesehen ist und vorwärts/rückwärts bewegt werden kann, um gemäß der Betätigung des Griffs (49) in Richtung einer zentralen Achse der Sonde (11) zwischen einer der geöffneten Position der Klaue (17) entsprechenden ersten Bewegungsposition und einer der geschlossenen Position der Klaue (17) entsprechenden zweiten Bewegungsposition bewegt zu werden;
ein Schieberaufnahmeelement (64), das in dem Gerätehauptkörper vorgesehen ist und dazu eingerichtet ist, die Bewegung des Schieberabschnitts (65) zu führen;
einen Federaufnehmer (69), der in einer Außenumfangsfläche des Schieberaufnahmeelements (64) angeordnet ist;
eine Spiralfeder (67), die zwischen dem Schieberabschnitt (65) und dem Federaufnehmer (69) angeordnet ist; und
einen Stopper, der dazu eingerichtet ist, mit dem Schieberaufnahmeelement (64) in Kontakt gebracht zu werden;
wobei, wenn der Schieberabschnitt (65) gemäß der Betätigung des Griffs (49) von der ersten Bewegungsposition in die zweite Position bewegt wird, auch das Schieberaufnahmeelement (64) gemeinsam mit dem Schieberabschnitt (65) vorwärts bewegt wird;
wenn die Betätigungskraft des Griffs (49) größer als ein vorbestimmter Betrag ist, das Schieberaufnahmeelement (64) gemäß der Betätigung des Griffs (49) in Kontakt mit dem Stopper gebracht wird und sich anschließend lediglich der Schieberabschnitt (65) gegen eine Federkraft der Spiralfeder (67) in eine distale Richtung bewegt, und anschließend, wenn eine Länge der Spiralfeder (67) entsprechend einer Bewegung des Schieberabschnitts (65) um einen festgelegten Betrag (S1) auf eine Länge (L2) geändert wird, wobei der festgelegte Betrag (S1) einer vorbestimmten Greifkraft zum Greifen von lebendem Gewebe durch die Klaue (17) entspricht, ein Anzeigemechanismus (801, 811) einen Zustand anzeigt, in dem sich der Schieberabschnitt (65) auf seinem Weg von der ersten Bewegungsposition in die zweite Bewegungsposition um einen Betrag bewegt hat, der gleich oder größer als der festgelegte Betrag (S1) ist.

2. Chirurgisches Operationsgerät gemäß Anspruch 1, wobei der Anzeigemechanismus (801, 811) einen Anzeigeabschnitt umfasst, der dazu eingerichtet ist, eine Anzeige durch ein Geräusch und/oder ein Klickgefühl durchzuführen.

3. Chirurgisches Operationsgerät gemäß Anspruch 2, wobei der Anzeigeabschnitt umfasst:
einen zylindrischen Körper (802), der an der Seite des Schieberaufnahmeelements (64) befestigt ist und an seiner Außenumfangsfläche mit einem Stufenabschnitt (802c) versehen ist, bei dem sich sein Durchmesser an einer Position, die dem festgelegten Betrag (S1) entspricht, in einer Bewegungsrichtung des Schieberabschnitts (65) verändert, und
ein Blattfederelement (803), das mit ersten und zweiten Endteilen (803b, 803d) versehen ist, wobei ein erstes Endteil (803b) an dem Schieberabschnitt (65) befestigt ist und das zweite Endteil (803d) an der Außenumfangsfläche des zylindrischen Körpers (802) in einem Zustand gehalten ist, in dem das zweite Endteil (803d) in Schiebekontakt mit der Außenumfangsfläche des zylindrischen Körpers (802) steht, und
zum Zeitpunkt der Bewegung des Schieberabschnitts (65), wenn das Blattfederelement (803) den Stufenabschnitt (802c) passiert, das zweite Endteil (803d) des Blattfederelements (803) dazu eingerichtet ist, über den Stufenabschnitt (802c) zu fallen und dazu eingerichtet ist, durch Schlagen der Oberfläche der Umfangswand (802a) an die Unterseite des Stufenabschnitts (802c) ein Schlaggeräusch zu erzeugen.

4. Chirurgisches Operationsgerät gemäß Anspruch 2, wobei der Anzeigeabschnitt ein Geräuscherzeugungselement (812) umfasst, das an dem Schieberaufnahmeelement (64) vorgesehen ist und dazu eingerichtet ist, ein Geräusch zu erzeugen, indem es in dem Zustand, in dem sich der Schieberabschnitt (65) um den Betrag, der gleich dem oder größer als der festgelegte Betrag (S1) ist, auf seinem Weg von der ersten Bewegungsposition in die zweite Bewegungsposition bewegt hat, in Kontakt mit dem Schieberabschnitt (65) gebracht wird.

5. Chirurgisches Operationsgerät gemäß Anspruch 1, wobei der festgelegte Betrag (S1) an einer Position festgelegt ist, die einem Betätigungsbetrag des Griffs (49) entspricht, wobei der Betätigungsbetrag die geeignete auszuübende Ultraschallbehandlungsfähigkeit ermöglicht, wenn das lebende Gewebe zwischen der Klaue (17) und dem distalen Endteil der Sonde (11) gegriffen ist.

6. Chirurgisches Operationsgerät gemäß Anspruch 5, wobei die Ultraschallbehandlungsfähigkeit Koagulation und/oder Schneiden ist.

## Revendications

1. Appareil de fonctionnement chirurgical comprenant :
une gaine (18) prévue avec une partie d'extrémité distale et une partie d'extrémité proximale ;
un corps principal d'appareil destiné à être couplé à la partie d'extrémité proximale de la gaine (18) ;
une sonde (11) qui est prévue avec une partie d'extrémité distale et une partie d'extrémité proximale, est insérée à l'intérieur de la gaine (18), et transmet des vibrations ultrasonores du côté de partie d'extrémité proximale au côté de partie d'extrémité distale ;
une mâchoire (17) qui est supportée tournante au niveau de la partie d'extrémité distale de la gaine (18), et peut être opérée de façon à être ouverte ou fermée entre une position fermée à laquelle la mâchoire (17) est engagée avec la partie d'extrémité distale (3a) de la sonde (11), et une position ouverte à laquelle la mâchoire (17) est séparée de la partie d'extrémité distale (3a) de la sonde (11) ;
une poignée (49) qui est prévue sur le corps principal d'appareil, et est adaptée à opérer l'opération d'ouverture/fermeture de la mâchoire (17) en fonction d'une force d'opération de la poignée (49) ;
une section de coulisse (65) qui est prévue dans le corps principal d'appareil, et peut être avancée/rétractée de façon à être déplacée dans un sens d'axe central de la sonde (11) entre une première position de mouvement correspondant à la position ouverte de la mâchoire (17) et une deuxième position de mouvement correspondant à la position fermée de la mâchoire (17) en fonction de l'opération de la poignée (49) ;
un élément (64) de réception de coulisse qui est prévu dans le corps principal d'appareil, et est adapté à guider le mouvement de la section de coulisse (65) ;
un récepteur (69) de ressort agencé sur une surface circonférentielle extérieure de l'élément (64) de réception de coulisse ;
un ressort hélicoïdal (67) étant agencé entre la section de coulisse (65) et le récepteur (69) de ressort ; et
une butée configurée pour être amenée en contact avec l'élément (64) de réception de coulisse ;
dans lequel, lorsque la section de coulisse (65) est déplacée de la première position de mouvement à la deuxième position en fonction de l'opération de la poignée (49), l'élément (64) de réception de coulisse est également avancé en même temps que la section de coulisse (65) ;
lorsque la force d'opération de la poignée (49) est supérieure à une quantité prédéterminée, l'élément (64) de réception de coulisse est amené en contact avec la butée, et, ensuite, seule la section de coulisse (65) se déplace dans un sens distal contre une force de ressort du ressort hélicoïdal (67) en fonction de l'opération de la poignée (49), et
ensuite, lorsqu'une longueur du ressort hélicoïdal (67) est changée jusqu'à une longueur (L2) correspondant à un mouvement de la section de coulisse (65) d'une quantité fixée (S1), dans lequel la quantité fixée (S1) correspond à une force de préhension prédéterminée pour capturer un tissu vivant par la mâchoire (17), un mécanisme de notification (801, 811) notifie d'un état où la section de coulisse (65) s'est déplacée d'une quantité égale ou supérieure à la quantité fixée (S1) sur le chemin de celle-ci de la première position de mouvement à la deuxième position de mouvement.

2. Appareil de fonctionnement chirurgical selon la revendication 1, dans lequel le mécanisme de notification (801, 811) inclut une section de notification qui est adapté à exécuter une notification au moyen d'un parmi un son et un ressenti d'un clic.

3. Appareil de fonctionnement chirurgical selon la revendication 2, dans lequel la section de notification inclut :
un corps cylindrique (802) qui est fixé sur le côté de l'élément (64) de réception de coulisse, et est prévu avec une section en marche (802c) sur une surface circonférentielle extérieure de celui-ci au niveau de laquelle un diamètre de celui-ci est changé à une position correspondant à la quantité fixée (S1) dans un sens de mouvement de la section de coulisse (65), et
un élément (803) de ressort à lames qui est prévu avec des première et deuxième parties d'extrémité (803b, 803d), dans lequel une première partie d'extrémité (803b) est fixée à la section de coulisse (65), et la deuxième partie d'extrémité (803d) est maintenue sur la surface circonférentielle extérieure du corps cylindrique (802) dans un état où la deuxième partie d'extrémité (803d) est en contact coulissant avec la surface circonférentielle extérieure du corps cylindrique (802), et
au moment du mouvement de la section de coulisse (65), lorsque l'élément (803) de ressort à lames passe la section en marche (802c), la deuxième partie d'extrémité (803d) de l'élément (803) de ressort à lames est adaptée à tomber sur la section en marche (802c), et est adaptée à générer un son de choc en heurtant la surface de paroi circonférentielle (802a) sur le côté inférieur de la section en marche (802c).

4. Appareil de fonctionnement chirurgical selon la revendication 2, dans lequel la section de notification inclut un élément (812) de génération de son qui est prévu sur l'élément (64) de réception de coulisse, et est adapté à générer le son en étant amené en contact avec la section de coulisse (65) dans l'état où la section de coulisse (65) s'est déplacée de la quantité égale ou supérieure à la quantité fixée (S1) sur le chemin de celle-ci de la première position de mouvement à la deuxième position de mouvement.

5. Appareil de fonctionnement chirurgical selon la revendication 1, dans lequel la quantité fixée (S1) est fixée à une position correspondant à une quantité de fonctionnement de la poignée (49), la quantité de fonctionnement permettant que la capacité de traitement par ultrasons appropriée soit exercée lorsque le tissu vivant est capturé entre la mâchoire (17) et la partie d'extrémité distale de la sonde (11).

6. Appareil de fonctionnement chirurgical selon la revendication 5, dans lequel la capacité de traitement par ultrasons est au moins une parmi une coagulation et une incision.
